# EUROPEAN PATENT SPECIFICATION

(11) **EP 4 198 028 B1**
(45) Date of publication and mention of the grant of the patent: **17.07.2024**
(21) Application number: 21214451.3
(22) Date of filing: 14.12.2021
(51) Int. Cl.: C07D 241/08

(54) **POLYTHIOL COMPOUNDS AND PROCESS FOR PREPARATION THEREOF**
POLYTHIOLVERBINDUNGEN UND VERFAHREN ZUR HERSTELLUNG DAVON
COMPOSÉS DE POLYTHIOL ET LEUR PROCÉDÉ DE PRÉPARATION

(43) Date of publication of application: 21.06.2023
(73) Proprietor: Henkel AG & Co. KGaA, 40589 Düsseldorf (DE)
(72) Inventor: Ito, Kenji, 40597 Düsseldorf (DE); Taden, Andreas, 40597 Düsseldorf (DE); Beck, Horst, 41470 Neuss (DE); Brandt, Adrian, 45219 Essen (DE)

(56) References cited:
- EP-A1- 3 760 665
- WO-A1-2019/183140
- HOYLE C E ET AL: "Thiol-enes: Chemistry of the past with promise for the future", JOURNAL OF POLYMER SCIENCE PART A: POLYMER CHEMISTRY, JOHN WILEY & SONS, INC, US, vol. 42, no. 21, 1 January 2004 (2004-01-01), pages 5301 - 5338, XP003024242, ISSN: 0887-624X, DOI: 10.1002/POLA.20366

## Description

### FIELD OF THE INVENTION

The present disclosure is directed to novel polythiol compounds which may be derived from bio-renewable resources. In particular, the present disclosure is directed to polythiol compounds which may be derived from the amino acids serine and tyrosine.

### BACKGROUND TO THE INVENTION

The chemical industry is currently reliant on a historically inexpensive, petroleum-based carbon feedstock that generates a small collection of platform chemicals from which highly efficient chemical conversions lead to the manufacture of a large variety of chemical products. The current approach to producing these carbon-based chemicals is, however, inherently non-sustainable as humans consume feedstock that required millions of years to form. The creation of a sustainable chemical industry will only occur when the timescale of feedstock formation equates to the timescale of its utilization in manufacturing chemicals.

Given the intrinsic need for carbon in chemical products, biomass - with its short formation time - must ultimately become the feedstock for the chemical industry. Biomass does represent an inexpensive feedstock but, if the chemical industry is to move to a biomass feedstock without a significant cost disruption, an efficient production paradigm will need to be developed that allows inexpensive and efficient processing of biomass.

Thiol-ene and thiol-epoxy polymers are attractive materials for coatings, adhesives and sealants due to their ease of fabrication and low shrinkage and stress. Moreover, the use of so-called *"click reactions"* in the synthesis of these materials provides an ecologically-friendly technology on account of the limited amount of waste originated, the low energy consumption, the completeness of the reaction process and the high yield without the formation of by-products that leads to the formation of homogenous three-dimensional network structures. In addition, such click reactions can be performed in the absence of solvent and in an air atmosphere - on account of the limited water and oxygen sensitivity of the reactants - which is of benefit to a number of technological applications.

Problematically, however, thiol-containing, ene-containing and epoxide compounds are traditionally derived from petrochemical sources. As such, certain authors have tried to derivatize compounds occurring naturally in biomass to enable their use in *inter alia* click reactions.

US2019/211139 (Robertson et al) discloses a resin comprising: (A) a naturally occurring aromatic compound wherein the aromatic compound has been modified to contain at least one epoxidized hydroxy group and at least one epoxidized carboxylic acid group; and (B) a curing agent selected from an anhydride, an acid, an alcohol, a thiol, a phenol, or an amine; provided that the aromatic compound is not an epoxidized version of gallic acid.

US2008/0021209A1A (East et al.) discloses a method for the synthesis of bisglycidyl ethers of anhydrosugars derived from renewable sources, including isosorbide, isomannide and isoidide. The bisglycidyl ethers are mooted to be useful substitutes for bisphenol A diglycidyl ethers. WO2010/136725A1 (Centre National de la Recherche Scientifique (C.N.R.S.) et al.) describes a process for preparing epoxy resins from a mixture of epoxidized phenolic compounds, wherein said epoxidized phenolic compounds are obtained by epoxidation of natural phenolic compounds selected from the group consisting of simple phenol, phenol acid, coumarin, naphthoquinone, stilbenoid, flavonoid, isoflavonoid, anthocyanin, condensed tannin and hydrolysable tannin.

US Patent No. 8,513,374 B2 (Dasgupta) describes -ene compounds which are derivatives of a plant polyphenol and which are selected from the group consisting of curcumin diallyl carbonate, tetrahydrocurcumin diallyl carbonate, resveratrol triallyl carbonate, mono-O-allyl curcumin, tetra-allyl curcumin, di-allyl tetrahydrocurcumin, tetra-allyl tetrahydrocurcumin and tri-O-allyl resveratrol. The utility of these allyl functionalized compounds as monomers in formation of polymers via thiol-ene chemistry is disclosed. EP 3 760 665, WO 2019/183140 and Hoyle C. et al.: "Thiol-enes: Chemistry of the past with promise for the future", Journal of Polymer Science Part A: Polymer Chemistry, vol. 42, no. 21, pages 5301-5338, refer to polythiol compounds and curable compositions thereof.

These citations do not however teach or suggest thiol functional compounds which may be derived from bio-renewable compounds. There is therefore considered to be a need in the art to provide such thiol functional compounds.

### STATEMENT OF THE INVENTION

In accordance with a first aspect of the invention, there is provided a polythiol compound having the general Formula VIA, VIB or VIC: 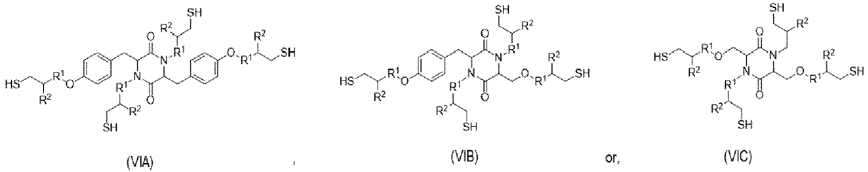 wherein:
R¹ is a C₁-C₁₈ alkylene group, C₂-C₁₈ oxyalkylene group, C₂-C₁₈ thioalkylene group,
C₆-C₁₈ arylene or C₇-C₁₈ aralkylene group; and,
R² is H or methyl.

As regards said general formulae, it is preferred that R¹ is an unsubstituted C₁-C₁₂ alkylene group, more preferably an unsubstituted C₁-C₄ alkylene group; and, R² is H or methyl.

Representative and important polythiols in accordance with these general formulae are selected from:

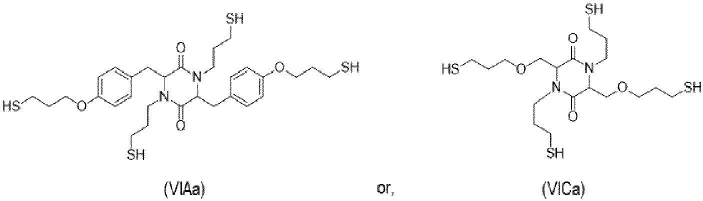

More particularly, the stereoisomers of compounds VIAa and VICa illustrated in Table 1 below represent important embodiments of the present disclosure.

**Table 1**

| Stereoisomer | Compound Name |
|---|---|
| 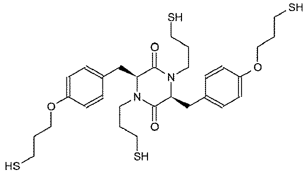 | (3S,6S)-3,6-bis[[4-(3-sulfanylpropoxy)phenyl]methyl]-1,4-bis(3-sulfanylpropyl)piperazine-2,5-dione |
| 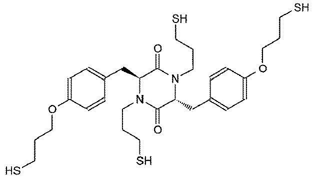 | (3R,6S)-3,6-bis[[4-(3-sulfanylpropoxy)phenyl]methyl]-1,4-bis(3-sulfanylpropyl)piperazine-2,5-dione |
| 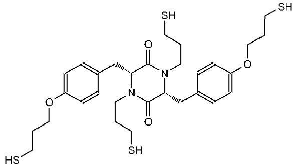 | (3R,6R)-3,6-bis[[4-(3-sulfanylpropoxy)phenyl]methyl]-1,4-bis(3-sulfanylpropyl)piperazine-2,5-dione |
| 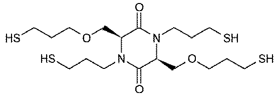 | (3S, 6S)-3,6-bis[(3-sulfanylpropoxy)methyl]-1,4-bis(3-sulfanylpropoxy)piperazine-2,5-dione |
| 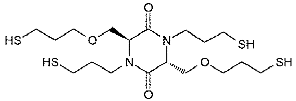 | (3R, 6S)-3,6-bis[(3-sulfanylpropoxy)methyl]-1,4-bis(3-sulfanylpropoxy)piperazine-2,5-dione |
| 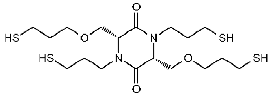 | (3R, 6R)-3,6-bis[(3-sulfanylpropoxy)methyl] - 1,4-bis(3-sulfanylpropoxy)piperazine-2,5-dione |

In accordance with a further aspect of the invention, there is provided a process for preparing a compound of Formula VIA, VIB or VIC as defined hereinabove and in the appended claims, said process comprising providing a compound having the general Formula IA, IB or IC: 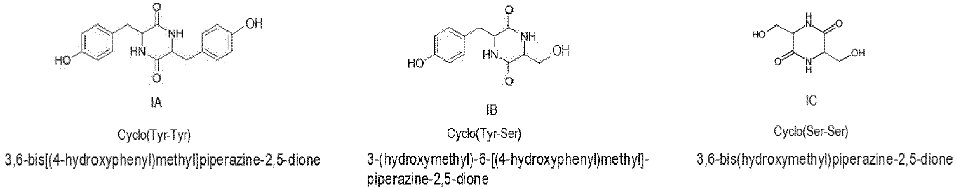 and further comprising the steps of
a) reacting, in the presence of at least one base, said compound of general Formula IA, IB or IC with a primary allyl compound (II) having the general formula:

   X-(R¹)-C(R²)=CH₂ (II)

   wherein:
   X is a halogen or an -OC(=O)OR° group;
   R° is a C₁-C₄ alkyl group;
   R¹ is a C₁-C₁₈ alkylene group, C₂-C₁₈ oxyalkylene group, C₂-C₁₈ thioalkylene group, C₆-C₁₈ arylene or C₇-C₁₈ aralkylene group; and,
   R² is H or methyl
   to form, respectively, a compound of Formula IIIA, IIIB or IIIC; 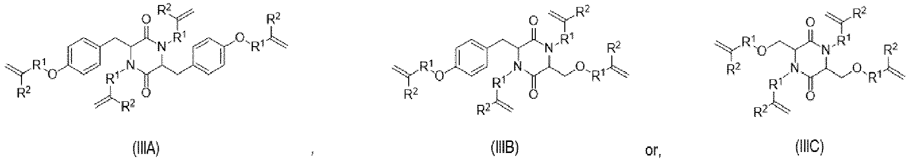
b) reacting said compound of Formula IIIA, IIIB or IIIC with a thiol acid (IV) having the general formula

   R³-C(=O)-SH (IV)

   wherein: R³ is C₁-C₆ alkyl, C₆-C₁₈ aryl, C₇-C₁₈ alkylaryl or C₇-C₁₈ aralkyl, to form, respectively, a thioester compound of Formula VA, VB or VC; 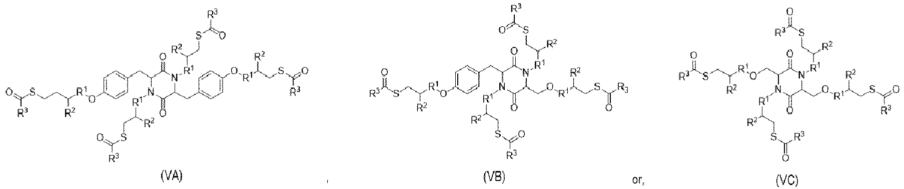 and,
c) deprotecting said thioester compound of Formula VA, VB or VC to form, respectively, said polythiol compound of Formula VIA, VIB or VIC.

In the described process, it is preferred that said primary allyl compound (II) is characterized in that: R° is C₁-C₂ alkyl; R¹ is an unsubstituted C₁-C₁₂ alkylene group, preferably an unsubstituted C₁-C₄ alkylene group; and, R² is H or Me. In important embodiments, said primary allyl compound (II) is selected from the group consisting of: 3-chloro-1-propene, 3-bromoprop-1-ene; 3-iodoprop-1-ene; methyl prop-2-enyl carbonate; and, ethyl prop-2-enyl carbonate.

As regards step b), it is preferred that said reactant thiol acid (IV) is characterized in that: R³ is C₁-C₄ alkyl, C₆ aryl, C₇-C₁₈ alkylaryl or C₇-C₁₈ aralkyl. A particular preference may be noted for said thiol acid (IV) being selected from the group consisting of thioacetic acid, thiopropionic acid; thiobutyric acid, thioisobutyric acid, thiobenzoic acid, 2-methyl-thiobenzoic acid, 3-methyl-thiobenzoic acid, 4-methyl-thiobenzoic acid, 2,4-dimethyl-thiobenzoic acid and 3,5-dimethyl-thiobenzoic acid. And good results have been obtained where either thioacetic or thiobenzoic acid is used as the reactant thiol acid (IV).

In accordance with an alternative aspect of the invention, there is provided a process for preparing a compound of Formula VIA, VIB or VIC as defined hereinabove and in the appended claims, said process comprising providing a compound having the general Formula IA, IB or IC: 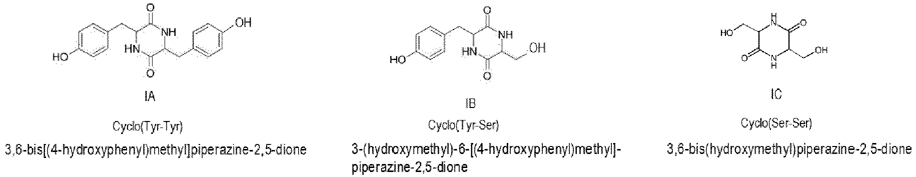 and further comprising the steps of:
α) reacting, in the presence of at least one base, said compound of general Formula IA, IB or IC with a thiolate ester compound having general Formula VII: 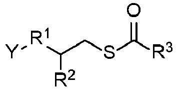 wherein:
   Y denotes a halogen;
   R¹ is a C₁-C₁₈ alkylene group, C₂-C₁₈ oxyalkylene group, C₂-C₁₈ thioalkylene group, C₆-C₁₈ arylene or C₇-C₁₈ aralkylene group;
   R² is H or methyl; and,
   R³ is C₁-C₆ alkyl, C₆-C₁₈ aryl, C₇-C₁₈ alkylaryl or C₇-C₁₈ aralkyl, to form, respectively, a thioester compound of Formula VA, VB or VC: 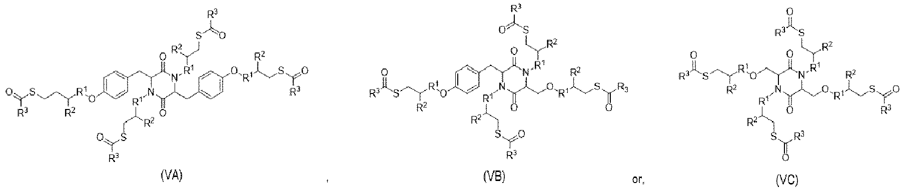
   and,
β) deprotecting said thioester compound of Formula VA, VB or VC to form, respectively, said polythiol compound of Formula VIA, VIB or VIC.

As regards said thiolate ester compound of Formula VII, it is preferred that: R¹ is an unsubstituted C₁-C₁₂ alkylene group; R² is H or Me; and, R³ is C₁-C₄ alkyl, C₆ aryl, C₇-C₁₈ alkylaryl or C₇-C₁₈ aralkyl. In important embodiments: R¹ is an unsubstituted C₁-C₄ alkylene group; R² is H or Me; and, R³ is a C₁-C₄ alkyl.

The deprotection step of the alternative processes above (c), β)) may be performed reductively but is preferably performed by acid-catalysed or base-catalysed hydrolysis.

The compounds of Formula IA, IB and IC can be provided from bio-renewable resources and, in particular from specific amino acids. Respectively, said compound of Formula IA may be provided by the homo-cyclization of tyrosine; said compound of Formula IB may be provided by the hetero-cyclization of tyrosine and serine; and, said compound of Formula IC may be provided by the homo-cyclization of serine.

In accordance with the further aspect of the disclosure there is provided the use of the polythiol compound as defined herein above and in the appended claims as a reactive component of an one (1K) or two (2K) component curable composition.

The disclosure also provides a curable composition comprising:
a) at least one polythiol as defined herein above and in the appended claims; and,
b) at least one thiol reactive compound, wherein the or each said thiol reactive compound has at least one functional group (F) selected from the group consisting of: epoxide groups; oxetane groups; cyclic carbonate groups; cyclic anhydride groups; 1-oxacycloalkan-2-one groups; ethylenically unsaturated groups; alkyne groups; and, isocyanate groups.

It will be recognized that, when compounds IA, IB and IC are sourced biorenewably, the resultant compounds will have a significant biocarbon content, wherein biocarbon content (%) is defined as: 100*(No. of Carbon Atoms of Biobased Starting Material (I) / No. of Carbon Atoms of Synthesized Polythiol). The following attainable biocarbon contents may be noted: Compound VlAa, 60%: and, Compound VICa, 33%.

### DEFINITIONS

As used herein, the singular forms "a", *"an"* and *"the"* include plural referents unless the context clearly dictates otherwise.

The terms *"comprising, "comprises"* and *"comprised* of" as used herein are synonymous with *"including", "includes", "containing"* or *"contains",* and are inclusive or open-ended and do not exclude additional, non-recited members, elements or method steps.

As used herein, the term *"consisting of'* excludes any element, ingredient, member or method step not specified. For completeness, the term *"comprising"* encompasses *"consisting of'.*

When amounts, concentrations, dimensions and other parameters are expressed in the form of a range, a preferable range, an upper limit value, a lower limit value or preferable upper and limit values, it should be understood that any ranges obtainable by combining any upper limit or preferable value with any lower limit or preferable value are also specifically disclosed, irrespective of whether the obtained ranges are clearly mentioned in the context.

Further, in accordance with standard understanding, a weight range represented as being *"from 0"* specifically includes 0 wt.%: the ingredient defined by said range may or may not be present in the composition.

The words *"preferred", "preferably", "desirably"* and *"particularly"* are used frequently herein to refer to embodiments of the disclosure that may afford particular benefits, under certain circumstances. However, the recitation of one or more preferable, preferred, desirable or particular embodiments does not imply that other embodiments are not useful and is not intended to exclude those other embodiments from the scope of the disclosure.

As used throughout this application, the word *"may"* is used in a permissive sense - that is meaning to have the potential to - rather than in the mandatory sense.

As used herein, room temperature (RT) is 23°C plus or minus 2°C. As used herein, *"ambient conditions"* means the temperature and pressure of the surroundings in which the composition is located or in which an adhesive, sealant, coating or composite structure obtained from said composition is located.

The molecular weights referred to in this specification - to describe to macromolecular, oligomeric and polymeric components of the curable compositions - can be measured with gel permeation chromatography (GPC) using polystyrene calibration standards, such as is done according to ASTM 3536.

The term *"molar equivalency"* is used in accordance with its standard meaning: it thus refers to the number of moles of specified compound or functional group A in relation to the number of moles of specified compound or functional group B.

As used herein, the term *"equivalent (eq.")* relates, as is usual in chemical notation, to the relative number of reactive groups present in the reaction.

The term *"equivalent weight* as used herein refers to the molecular weight divided by the number of a function concerned. As such, *"epoxide equivalent weight*" (EEW) means the weight of resin, in grams, that contains one equivalent of epoxide.

As used herein, the term *"water"* is intended to encompass tap water, spring water, purified water, de-ionized water, de-mineralized and distilled water.

As used herein *"basicity"* means the quality of being a base, not an acid. More particularly, in accordance with the Lewis theory of acids and bases, a base is an electron-pair donor. This definition encompasses but is not limited to Bronsted-Lowry bases, which compounds act as proton acceptors.

As used herein, the term *"one component (1K) composition"* refers to a composition where, during storage of the composition, the composition components are all admixed together but the properties of the composition, including viscosity, remain consistent enough over the time of storage to permit successful utility of the composition at a later time.

*"Two-component (2K) compositions"* are understood to be compositions in which a first component (A) and a second component (B) must be stored in separate vessels because of their (high) reactivity. The two components are mixed only shortly before application and then react, typically without additional activation, with bond formation and thereby formation of a polymeric network. Herein higher temperatures may be applied in order to accelerate the cross-linking reaction.

Certain embodiments of the present disclosure describe dual cure compositions. As used herein, the term *"dual cure composition"* refers to a composition that will cure upon exposure to two different cure conditions. For example, the dual cure compositions may cure upon exposure to a combination of thermal energy and radiation. Thermal energy is intended to encompass: radiant energy, such as infrared or microwave energy; and, conductive thermal energy, such as that produced by a heated plate or an oven. As used herein, the term *"radiation"* encompasses: ionizing radiation, such as an electrons beam; and, actinic radiation.

As used herein, the term *"free radical initiator"* refers to any chemical species which, upon exposure to sufficient energy - in the form of light or heat, for example - decomposes into two parts which are uncharged, but which each possess at least one unpaired electron. Thus a thermal free radical initiator generates free upon exposure to heat. And known thermal free radical initiators include, but are not limited to, peroxide compounds, azo compounds and persulfate compounds.

The term "*photoinitiator*" as used herein denotes a compound which can be activated by an energy-carrying activation beam - such as electromagnetic radiation - upon irradiation therewith. Specifically, a *"free-radical photoinitiator"* herein refers to a photoactive compound that generates free radicals, which radicals could herein initiate polymerization or reaction by addition to C=C double bonds present in the compositions.

The term *"aprotic solvents"* as used herein refers to solvents that do not yield or accept a proton. Conversely *"protic solvents"* are those solvents capable of yielding or accepting a proton. The *"polar solvent'* as used herein refers to a solvent having a dielectric constant (ε) of more than 5 as measured at 25°C: the term encompasses both aprotic and protic solvents. The determination of dielectric constant (ε) is well known in the art and is within the knowledge of the skilled person: the use of measured voltages across parallel plate capacitors in such determinations may be mentioned.

As used herein, the term "epoxide" denotes a compound characterized by the presence of at least one cyclic ether group, namely one wherein an ether oxygen atom is attached to two adjacent carbon atoms thereby forming a cyclic structure. The term is intended to encompass monoepoxide compounds, polyepoxide compounds (having two or more epoxide groups) and epoxide terminated prepolymers. The term *"monoepoxide compound"* is meant to denote epoxide compounds having one epoxy group. The term *"polyepoxide compound"* is meant to denote epoxide compounds having at least two epoxy groups. The term *"diepoxide compound"* is meant to denote epoxide compounds having two epoxy groups.

The epoxide may be unsubstituted but may also be inertly substituted. Exemplary inert substituents include chlorine, bromine, fluorine and phenyl.

As used herein, "*(meth)acryf*" is a shorthand term referring to "*acryl*" and/or "*methacryl*". Thus the term *"(meth)acrylate"* refers collectively to acrylate and methacrylate. Likewise, "*allyl*" and "*methallyl*" are designated herein in a summarizing manner as "*(meth)allyl*"*.*

As used herein, "*C₁*-*Cₙ alkyl*" group refers to a monovalent group that contains 1 to n carbons atoms, that is a radical of an alkane and includes straight-chain and branched organic groups. As such, a "*C₁*-*C₁₈ alkyl*" group refers to a monovalent group that contains from 1 to 18 carbons atoms, that is a radical of an alkane and includes straight-chain and branched organic groups. Examples of alkyl groups include, but are not limited to: methyl; ethyl; propyl; isopropyl; n-butyl; isobutyl; sec-butyl; tert-butyl; n-pentyl; n-hexyl; n-heptyl; and, 2-ethylhexyl. In the present invention, such alkyl groups may be unsubstituted or may be substituted with one or more halogen. Where applicable for a given moiety (R), a tolerance for one or more non-halogen substituents within an alkyl group will be noted in the specification.

The term "*C₁*-*Cₙ hydroxyalkyl*" as used herein refers to a HO-(alkyl) group having from 1 to n carbon atoms, where the point of attachment of the substituent is through the oxygen-atom and the alkyl group is as defined above.

An *"alkoxy group"* refers to a monovalent group represented by -OA where A is an alkyl group: non-limiting examples thereof are a methoxy group, an ethoxy group and an iso-propyloxy group. The term "*C₁-C₁₂ alkoxyalkyl*" as used herein refers to an alkyl group having an alkoxy substituent as defined above and wherein the moiety (*alkyl-O-alkyl*) comprises in total from 1 to 12 carbon atoms: such groups include methoxymethyl (-CH₂OCH₃), 2-methoxyethyl (-CH₂CH₂OCH₃) and 2-ethoxyethyl.

As used herein, the term "*C₁*-*C₈ alkanol"* refers to compounds of the general formula ROH, where R is an alkyl group having from 1 to 8 carbon atoms as defined above. In the described method steps, a preference for a C₁-C₄ alkanol and more particularly for methanol or ethanol should be noted.

The term "*C₁*-*Cₙ alkylene"* as used herein, is defined as saturated, divalent hydrocarbon radical having from 1 to n carbon atoms. In general in the present disclosure, such alkylene groups may be unsubstituted or may be substituted with one or more halogen or hydroxyl. Where applicable for a given moiety (R), a tolerance for one or more non-halogen substituents within an alkylene group will be noted in the specification.

By the term "*C₂*-*C₁₈ oxyalkylene group"* is meant two or more alkylene groups linked by one or more oxygen atoms in ether linkage, said group having in total from 2 to 19 carbon exams. Examples thereof include mono-ether groups being represented by *-alkylene-O-alkylene-* and di-ether groups being represented by *-alkylene-O-alkylene-O-alkylene-,* wherein the term alkylene has the meaning assigned above.

By the term "*C₂*-*C₁₈ thioalkylene group"* is meant two or more alkylene groups linked by one or more sulphur atoms in thioether linkage, said group having in total from 2 to 19 carbon exams. Examples thereof include mono-thioether groups being represented by *-alkylene-S-alkylene-* and di-thioether groups being represented by *-alkylene-S-alkylene-S-alkylene-,* wherein the term alkylene has the meaning assigned above.

The term "*C₃* -*C₃₀ cycloalkyl*" is understood to mean a saturated, mono- or polycyclic hydrocarbon group having from 3 to 30 carbon atoms. In the present invention, such cycloalkyl groups may be unsubstituted or may be substituted with one or more halogen. Where applicable for a given moiety (R), a tolerance for one or more non-halogen substituents within a cycloalkyl group will be noted in the specification. Examples of cycloalkyl groups include: cyclopropyl; cyclobutyl; cyclopentyl; cyclohexyl; cycloheptyl; cyclooctyl; adamantane; and, norbornane.

The term "*C₃-C₃₀ hydroxycycloalkyl*" as used herein refers to a HO-(cycloalkyl) group having from 3 to 30 carbon atoms, where the point of attachment of the substituent is through the oxygen-atom and the cycloalkyl group is as defined above.

As used herein, an "*C₆-C₁₈ aryl*" group used alone or as part of a larger moiety - as in *"aralkyl group"* - refers to monocyclic, bicyclic and tricyclic ring systems in which the monocyclic ring system is aromatic or at least one of the rings in a bicyclic or tricyclic ring system is aromatic. The bicyclic and tricyclic ring systems include benzofused 2-3 membered carbocyclic rings. In the present invention, such aryl groups may be unsubstituted or may be substituted with one or more halogen. Where applicable for a given moiety (R), a tolerance for one or more non-halogen substituents within an aryl group will be noted in the specification. Exemplary aryl groups include: phenyl; indenyl; naphthalenyl, tetrahydronaphthyl, tetrahydroindenyl; tetrahydroanthracenyl; and, anthracenyl. And a preference for phenyl groups may be noted.

The term "*C₆-C₁₈ arylene*" denotes a divalent hydrocarbon radical having from 6 to 18 carbon atoms which is derived from an aryl group as defined above. An exemplary arylene group is phenylene (-C₆H₄-).

As used herein, "*C₂-C₂₀ alkenyl*" refers to hydrocarbyl groups having from 2 to 20 carbon atoms and at least one unit of ethylenic unsaturation. The alkenyl group can be straight chained, branched or cyclic and may optionally be substituted with one or more halogen. Where applicable for a given moiety (R), a tolerance for one or more non-halogen substituents within an alkenyl group will be noted in the specification. The term *"alkenyl"* also encompasses radicals having *"cis"* and *"trans"* configurations, or alternatively, "E" and "Z" configurations, as appreciated by those of ordinary skill in the art. Examples of said C₂-C₂₀ alkenyl groups include, but are not limited to: -CH=CH₂; - CH=CHCH₃; -CH₂CH=CH₂; -C(=CH₂)(CH₃); -CH=CHCH₂CH₃; -CH₂CH=CHCH₃; - CH₂CH₂CH=CH₂; -CH=C(CH₃)₂; -CH₂C(=CH₂)(CH₃); -C(=CH₂)CH₂CH₃; -C(CH₃)=CHCH₃; - C(CH₃)CH=CH₂; -CH=CHCH₂CH₂CH₃; -CH₂CH=CHCH₂CH₃, -CH₂CH₂CH=CHCH₃; - CH₂CH₂CH₂CH=CH₂; -C(=CH₂)CH₂CH₂CH₃; -C(CH₃)=CHCH₂CH₃; -CH(CH₃)CH=CHCH_{;} - CH(CH₃)CH₂CH=CH₂; -CH₂CH=C(CH₃)₂; 1-cyclopent-1-enyl; 1-cyclopent-2-enyl; 1-cyclopent-3-enyl; 1-cyclohex-1-enyl; 1-cyclohex-2-enyl; and, 1-cyclohexyl-3-enyl.

As used herein, "*alkylaryl*" refers to alkyl-substituted aryl groups, both groups being defined as above. Further, as used herein "*aralkyl*" means an alkyl group substituted with an aryl radical as defined above. The term "*C₁*-*Cₙ aralkylene"* as used herein is defined as a saturated, divalent hydrocarbon radical having from 1 to n carbon atoms in which an alkylene group as defined above is substituted with an aryl radical as defined above.

The term *"hetero"* as used herein refers to groups or moieties containing one or more heteroatoms, such as N, O, Si and S. Thus, for example *"heterocyclic"* refers to cyclic groups having, for example, N, O, Si or S as part of the ring structure. "*Heteroalkyl*"*,* "*heterocycloalkyl*" and "*heteroaryl*" moieties are alkyl, cycloalkyl and aryl groups as defined hereinabove, respectively, containing N, O, Si or S as part of their structure.

As regard the compositions and process conditions which may be defined, the term *"substantially free"* is intended to mean that a compound, element, ion or other like component is not deliberately added to the composition or reaction mixture and is present, at most, in only trace amounts which will have no (adverse) effect on the desired properties of the composition or the progress of the reaction. An exemplary trace amount is less than 1000 ppm by weight of the composition. The term *"substantially free"* encompasses those embodiments where the specified compound, element, ion, or other like component is completely absent from the composition or reaction mixture or is not present in any amount measurable by techniques generally used in the art.

The term *"anhydrous"* as used herein has equivalency to the term *"substantially free of water*"*.* Water is not deliberately added to a given composition and is present, at most, in only trace amounts which will have no (adverse) effect on the desired properties of the composition.

### DETAILED DESCRIPTION OF THE INVENTION

The individual steps of the process as defined above will be discussed in more detail herein below. If a chemical compound is referred to using both a chemical structure and a chemical name, and an ambiguity exists between the structure and the name, the structure predominates.

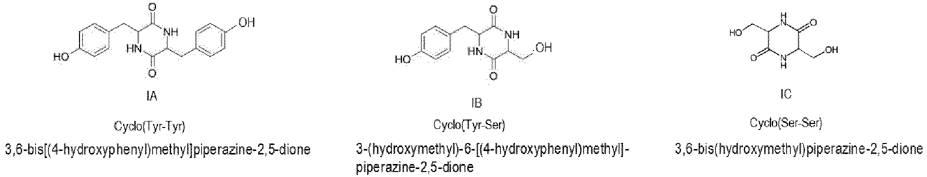

The compounds of Formula IA (3,6-bis[(4-hydroxyphenyl)methyl]piperazine-2,5-dione), IB (3-(hydroxymethyl)-6-[(4-hydroxyphenyl)methyl]piperazine-2,5-dione and IC (3,6-bis(hydroxymethyl)piperazine-2,5-dione) contain asymmetric or chiral centers and therefore exist in different stereoisomeric forms. The orientation of the amino acid lateral chains could be on the same side of the ring plane for the homochiral piperazine-2,5-dione stereoisomers (LL, DD) or on the opposite side for the heterochiral stereoisomers (LD or DL). It is intended that all stereoisomeric forms of the compounds of Formula IA to IC as well as mixtures thereof, including racemic mixtures, form part of the disclosure.

The compounds of Formulae IA to IC may be obtained commercially. In the alternative, the 2,5-diketopiperazines (2,5-DKPs) may be derived from the *"head to taif'* cyclization of two α-amino acids, specifically serine (Ser, 2-Amino-3-hydroxypropanoic acid) and tyrosine (Tyr, 2-Amino-3-(4-hydroxyphenyl)propanoic acid). Industrially, L-serine is conventionally produced from glycine and methanol catalysed by hydroxymethyltransferase. Tyrosine is conventionally produced by: extraction from protein hydrolysates; enzymatic synthesis from phenolics, pyruvate, and ammonia through the use of tyrosine phenol-lyase; or, industrial fermentation employing, in particular, productions strains of E. *coli.*

There is no intention to limit the method by which such cyclization may be performed. Mention in this regard may be made of: aza-Wittig cyclization; C-terminally protected dipeptide cyclization, typically wherein the C-terminal protecting group is a methyl ester such that the N-terminal amine acts as the nucleophile and the methoxy functionality is the leaving group; microwave assisted cyclization; and, enzymatic cyclization for which illustrative enzymes include cyclodipeptide synthases, oxidases and methyltransferases. Instructive disclosures in this regard include: Scarel et al. Diketopiperazine Gels: New Horizons from the Self Assembly of Cyclic Dipeptides Molecules 26: 3376 (2021); and, Peng et al. Renewable protein-based monomer for thermosets: a case study on phthalonitrile resin, Green Chemistry 20: 5158-5168 (2018), which document provides for protection-free cyclization of tyrosine.

In the description below, reference will be made to the appended drawings in which:
Figure 1 is illustrative of Step a) of the process of a first embodiment of the present disclosure;
Figure 2 is illustrative of Step b) of the process of a first embodiment of the present disclosure;
Figure 3 is illustrative of Step c) of the process of a first embodiment of the present disclosure; and,
Figure 4 is illustrative of Step α) of the process of a second embodiment of the present disclosure.

Further, in the description below, where reference is made to Formula I, III, V and VI, this is intended to denote, respectively, each of IA-IC, IIIA-IIIC, VA-VC and VIA-VIC.

### FIRST PROCESS EMBODIMENT

This process embodiment is characterized in that the compound of formula VI is formed from a compound of Formula I via an allyl functional intermediate (III).

### Step a): Allylation

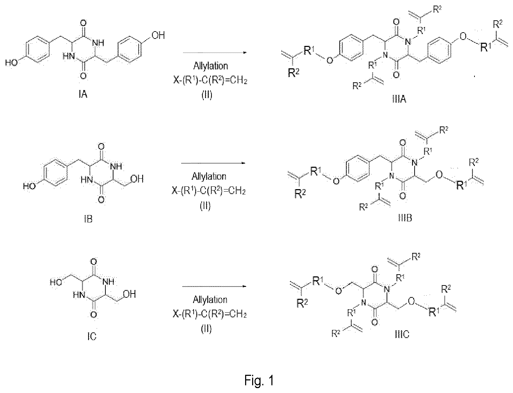

In this step of the described process, depicted in Figure 1, there is provided the reaction of a compound of Formula I with a primary allyl compound (II) in the presence of at least one base. The primary allyl compound (II) corresponds to the allyl substituent that is to be introduced into the allyl ether product (III) of this step. As denoted in the above reaction scheme, the primary allyl compound (II) has the general formula:

X-(R¹)-C(R²)=CH₂

wherein:
X is a halogen or an -OC(=O)OR° group;
R° is a C₁-C₄ alkyl group;
R¹ is a C₁-C₁₈ alkylene group, C₂-C₁₈ oxyalkylene group, C₂-C₁₈thioalkylene group, C₆-C₁₈ arylene or C₇-C₁₈ aralkylene group; and,
R² is H or methyl.

In most cases, allyl chloride (X=CI) is favoured because of lower cost, but the allyl bromides and allyl iodides may be more reactive, particularly in the case of high molecular weight allyl compounds. In an initial statement of preference, it is preferred that: R° is a C₁-C₄ alkyl; R¹ is an C₁-C₁₈ alkylene group, C₂-C₁₈ oxyalkylene group or C₂-C₁₈ thioalkylene group; and, R² is H or Me. In particular, it is preferred that: R° is a C₁-C₂ alkyl; R¹ is an unsubstituted C₁-C₁₂ alkylene group; and, R² is H or Me. In important embodiments: R¹ is an unsubstituted C₁-C₄ alkylene group; and, R² is H.

Exemplary primary allyl halides in accordance with Formula II include: 3-chloro-1-propene (*allyl chloride*); 3-bromoprop-1-ene (*allyl bromide*); 3-iodoprop-1-ene (*allyl iodide*); 3-chloro-2-methyl-1-propene (*methallyl chloride*); 3-bromo-2-methyl-1-propene (*methallyl bromide*); 3-iodo-2-methyl-1-propene (*methallyl iodide*); 4-chloro-but-1-ene; 4-bromo-but-1-ene; 4-iodo-but-1-ene; 5-chloropent-1-ene; 5-bromopent-1-ene; 5-iodopent-1-ene; 6-chlorohex-1-ene; 6-bromohex-1-ene; 6-iodohex-1-ene; 7-chlorohept-1-ene; 7-bromohept-1-ene; 7-iodohept-1-ene; 8-chlorooct-1-ene; 8-bromooct-1-ene; 8-iodooct-1-ene; 10-chlorodec-1-ene; 10-bromodec-1-ene; 10-iododec-1-ene; 12-chlorododec-1-ene; 12-bromododec-1-ene; and, 12-iododoc-1-ene. Of the primary allyl halides, a preference for the use of 3-chloro-1-propene (*allyl chloride*); 3-bromoprop-1-ene (*allyl bromide*); 3-iodoprop-1-ene (*allyl iodide*) may be noted, and a particular preference for the use of 3-bromoprop-1-ene is acknowledged.

Exemplary further reactants in accordance with Formula II include: methyl prop-2-enyl carbonate; ethyl prop-2-enyl carbonate; prop-2-enyl propyl carbonate; n-butyl prop-2-enyl carbonate; methyl 2-methylprop-2-enyl carbonate; and, ethyl 2-methylprop-2-enyl carbonate. Of these reactants, a preference may be noted for the use of methyl prop-2-enyl carbonate or ethyl prop-2-enyl carbonate.

It is intended that each hydroxyl group and each amine group in the compounds of Formula I be subjected to allylation and, as such, the primary allyl compound (II) should be present in at least a stoichiometric equivalent amount to the total number of moles of said hydroxyl and amine groups. The reaction mixture may, in particular, include said primary allyl compound (II) at a molar equivalency of from 1 to 2 relative to the total number of moles of the amine and hydroxyl groups of Formula I. Preferably, the allyl compound (II) is reacted at a molar equivalency of from 1.2 to 1.8 relative to the total number of moles of the amine and hydroxyl groups of Formula I.

The base which is present will react with the halogen acid or the C₁-C₄ alkyl carbonic acid liberated in the reaction and therefore should be present in at least the stoichiometric amount from that reaction. The reaction is operable in the presence of excess base however and therefore the total amount of base present in the reaction mixture may be from 1 to 5 molar equivalents to the amount of compound (I). It is preferred that the total amount of base is from 1.5 to 3 molar equivalents or from 1.5 to 2.5 molar equivalents to the amount of compound (I).

Without intention to limit the present invention, the base should desirably consist of at least one compound selected from alkali metals, alkaline earth metals, alkali metal (C₁-C₄)alkoxides, alkaline earth metal (C₁-C₄) alkoxides, alkali metal carbonates, alkaline earth metal carbonates, alkali metal hydroxides, alkaline earth metal hydroxides, alkali metal hydrides and alkaline earth metal hydrides. For allylation at the phenolic hydroxyl group, the use of at least one of potassium carbonate, sodium carbonate and calcium carbonate may be preferred: stronger bases may however be required for allylation at the constituent -NH groups. Given this, the present disclosure does not preclude the performance of the allylation in two sub-steps, wherein in a first sub-step allylation is effected at the phenolic hydroxyl groups under a first set of basic conditions and subsequently, in a second sub-step, allylation is effected at the remaining ring -NH groups of compound (I) under a second set of basic conditions. The separation and purification of the intermediate formed in the first sub-step, prior to the second sub-step, is not precluded in this embodiment.

In certain embodiments, the addition of a neutral alkali metal salt to the reaction mixture can have a positive effect on the yield of the allyation product. Exemplary alkali metal salts include: sulfates; sulfites; sulfides; halides; nitrates; nitrites; cyanides; borates; phosphates; monohydrogen phosphates; dihydrogen phosphates; phosphites; acid phosphites; and, carboxylates, such as acetates, formates, propionates, oxalates, tartrates, succinates, maleates and adipates.

A small amount of the neutral alkali metal salt - for instance from 0.01 to 0.05 moles per mole of compound (I) - will have a yield-enhancing effect. The amount of said neutral salt should not however exceed the amount of base included in the reaction mixture.

This allylation step is preferably performed in the presence of inert aprotic solvent. Examples of suitable aprotic solvents, which may be used alone or in combination, include but are not limited to: pentane; hexane; heptanes; cyclopentane; cyclohexane; cycloheptane; dimethylether; chloroform; dimethyl carbonate; ethylmethyl carbonate; diethyl carbonate; toluene; o-xylene; m-xylene; p-xylene; ethylbenzene; 2-propylbenzene (cumene); 2-isopropyltoluene (*o-cymol*); 3-isopropyltoluene (m-*cymol*); 4-isopropyltoluene (*p-cymol*); 1,3,5-trimethylbenzene (*mesitylene*); acetonitrile; N,N-di(C₁-C₄)alkylacylamides, such as N,N-dimethylformamide (DMF) and N,N-dimethylacetamide (DMAc); hexamethylphosphoramide; N-methylpyrrolidone; pyridine; esters, such as (C₁-C₈)alkyl acetates, ethoxydiglycol acetate, dimethyl glutarate, dimethyl maleate, dipropyl oxalate, ethyl lactate, benzyl benzoate, butyloctyl benzoate and ethylhexyl benzoate; ketones, such as acetone, ethyl ketone, methyl ethyl ketone (*2*-*butanone*) and methyl isobutyl ketone; ethers, such as tetrahydrofuran (THF), 2-methyltetrahydrofuran (2-MeTHF) and 1,2-dimethoxyethane; 1,3-dioxolane; dimethylsulfoxide (DMSO); and, dichloromethane (DCM).

Whilst it is not critical, it is preferred that the reaction of this step be performed under anhydrous conditions. Where necessary, exposure to atmospheric moisture may be avoided by providing the reaction vessel with an inert, dry gaseous blanket. Whilst dry nitrogen, helium and argon may be used as blanket gases, precaution should be used when common nitrogen gases are used as a blanket, because such nitrogen may not be dry enough on account of its susceptibility to moisture entrainment; the nitrogen may require an additional drying step before use herein.

The reaction need not be performed under reflux conditions, but it is preferred to do so. Where reflux conditions are not used, the performance of the reaction at room temperature is not actually precluded but the use of elevated temperatures, for example at least 50°C or at least 75°C, can drive the reaction. For an exothermic reaction, some cooling might however be required as the reaction progresses.

The process pressure is not critical: as such, the reaction can be run at sub-atmospheric, atmospheric, or super-atmospheric pressures but pressures at or slightly above atmospheric pressure are preferred. Mention in this regard may be made of pressures of from 50 to 200 kPa, for example from 100 to 200 kPa.

The progress of the above reaction may be monitored by known techniques of which mention may be made of ¹H NMR, Fourier Transform Infrared Spectroscopy, Ultra Performance Liquid Chromatography (UPLC) or thin layer chromatography (TLC). Upon completion of the reaction, the obtained mixture is filtered to remove solids: the solvent is removed from the filtrate to obtain the crude product (III).

The crude product may be used in the subsequent step of the process or, alternatively, the relevant compound may be purified using methods known in the art, including but not limited to solvent extraction, filtration and chromatography.

### Step b) Thiol-ene Reaction

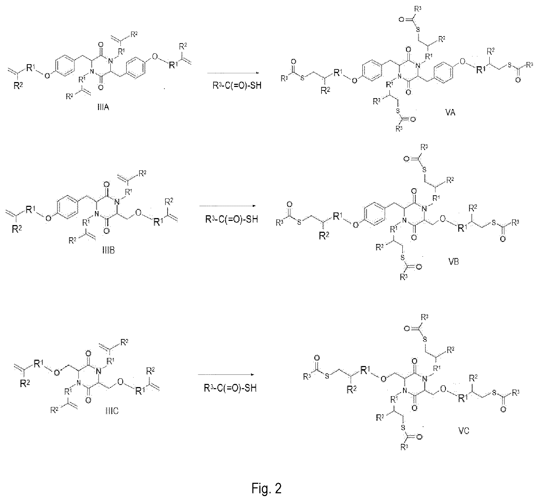

In this step, depicted in Figure 2, the allyl functional compound (III) of step a) is reacted with a thiol acid (IV). The reactant thiol acid (IV) has the general formula:

R³-C(=O)-SH

wherein: R³ is C₁-C₆ alkyl, C₆-C₁₈ aryl, C₇-C₁₈ alkylaryl or C₇-C₁₈ aralkyl.

Preferably R³ is C₁-C₄ alkyl, C₆ aryl, C₇-C₁₈ alkylaryl or C₇-C₁₈ aralkyl.

Exemplary thiol acids include: thioacetic acid; thiopropionic acid; thiobutyric acid; thioisobutyric acid; thiocinnamic acid (*3-phenylprop-2-enethioic S-acid*); thiobenzoic acid; 2-methyl-thiobenzoic acid; 3-methyl-thiobenzoic acid; 4-methyl-thiobenzoic acid; 2,4-dimethyl-thiobenzoic acid; and, 3,5-dimethyl-thiobenzoic acid. A particular preference for the use of thioacetic acid or thiobenzoic acid may be mentioned.

Where it is intended that the thiol-ene should occur at each allyl group, the thiol acid (IV) should be present in the reaction mixture in at least a stoichiometric equivalent amount to the moles of said allyl groups. The reaction mixture may, in this instance, include said thiol acid (IV) at a molar equivalency of from 1 to 2 moles relative to said allyl groups. Preferably, the amount of thiol acid (IV) is within the range from 1.2 to 1.8 moles per mole of allyl groups.

As the reactant includes more than one allyl group, the partial conversion of these groups may occur by using a sub-stoichiometric amount of thiol acid (IV) relative to the total number of moles of said allyl groups.

The skilled artisan will be aware that the reaction of thiol and -ene groups can occur under exposure to actinic irradiation and thus catalysts or accelerators may not strictly be required for this step of the process. However, this does not preclude the use in this step of a Michael addition catalyst, which herein refers to a compound capable of promoting a Michael addition reaction between the thiol acid and compounds of Formula III having terminal C=C unsaturation. Michael addition catalysts may be employed in an amount of from 0 to 5 wt.%, for example from 0.05 to 2 wt.%, based on the weight of said thiol acid.

Conventionally, Michael addition catalysts include amine-based catalysts, base catalysts and organometallic catalysts, which types may be used alone or in combination. Exemplary amine-based catalysts include: proline; triazabicyclodecene (TBD); diazabicycloundecene (DBU); hexahydro methyl pyrimido pyridine (MTBD); diazabicyclononane (DBN); tetramethylguanidine (TMG); and, triethylenediamine (TED, *1,4-diazabicyclo[2.2.2]octane).* Examples of the base catalysts include: sodium methoxide; sodium ethoxide; potassium t-butoxide; potassium hydroxide; sodium hydroxide; sodium metal; lithium diisopropylamide (LDA); and, butyllithium. Exemplary organometallic catalysts include: ruthenium catalysts such as (cyclooctadiene)(cyclooctatriene)ruthenium and ruthenium hydride; iron catalysts such as iron(III)chloride and iron acetylacetonate; nickel catalysts such as nickel acetylacetonate, nickel acetate and nickel salicylaldehyde; copper catalysts; palladium catalysts; scandium catalysts; lanthanum catalysts; ytterbium catalysts; and, tin catalysts.

Whilst the thiol-ene reaction can be performed under Michael addition catalysis, these reactions can also be initiated by a free radical generating thermal initiator or a free radical photoinitiator. In this embodiment, the reaction mixture of this step may include from 0.1 to 1 wt.%, for example from 0.1 to 0.5 wt.% of at least one free radical initiator, based on the weight of said thiol acid .

Typically, free radical photoinitiators are divided into those that form radicals by cleavage, known as "Norrish Type I", and those that form radicals by hydrogen abstraction, known as "Norrish Type II". The Norrish Type II photoinitiators require a hydrogen donor, which serves as the free radical source: as the initiation is based on a bimolecular reaction, the Norrrish Type II photoinitiators are generally slower than Norrish Type I photoinitiators which are based on the unimolecular formation of radicals. On the other hand, Norrish Type II photoinitiators possess better optical absorption properties in the near-UV spectroscopic region. The skilled artisan should be able to select an appropriate free radical photoinitiator based on the radiation being employed and the sensitivity of the photoinitiator(s) at that wavelength.

Preferred free radical photoinitiators are those selected from the group consisting of: benzoylphosphine oxides; aryl ketones; benzophenones; hydroxylated ketones; 1-hydroxyphenyl ketones; ketals; and, metallocenes. For completeness, the combination of two or more of these photoinitiators is not precluded in the present invention.

Particularly preferred free radical photoinitiators are those selected from the group consisting of: benzoin dimethyl ether; 1-hydroxycyclohexyl phenyl ketone; benzophenone; 4-chlorobenzophenone; 4-methylbenzophenone; 4-phenylbenzophenone; 4,4'-bis(diethylamino) benzophenone; 4,4'-bis(N,N'-dimethylamino) benzophenone (Michler's ketone); isopropylthioxanthone; 2-hydroxy-2-methylpropiophenone (Daracur 1173); 2-methyl-4-(methylthio)-2-morpholinopropiophenone; methyl phenylglyoxylate; methyl 2-benzoylbenzoate; 2-ethylhexyl 4-(dimethylamino)benzoate; ethyl 4-(N,N-dimethylamino)benzoate; phenylbis(2,4,6-trimethylbenzoyl)phosphine oxide; diphenyl(2,4,6-trimethylbenzoyl)phosphine oxide; and, ethyl phenyl(2,4,6-trimethylbenzoyl)phosphinate. Again, for surety, the combination of two or more of these photoinitiators is not precluded.

Where the reaction mixture of step b) comprises a free radical photoinitiator, irradiation of the reaction mixture generates the active species from the photoinitiator(s) which initiates the reactions. Once that species is generated, the chemistry is subject to the same rules of thermodynamics as any chemical reaction: the reaction rate may be accelerated by heat.

The energy source used to initiate the reaction of this embodiment of step b) will emit at least one of ultraviolet (UV) radiation, infrared (IR) radiation, visible light, X-rays, gamma rays, or electron beams (e-beam). The reaction may typically be activated in less than 2 minutes, and commonly between 0.1 and 100 seconds - for instance between 3 and 12 seconds - when irradiated using commercial curing equipment.

Irradiating ultraviolet light should typically have a wavelength of from 150 to 600 nm and preferably a wavelength of from 200 to 450 nm. Useful sources of UV light include, for instance, extra highpressure mercury lamps, high pressure mercury lamps, medium pressure mercury lamps, low intensity fluorescent lamps, metal halide lamps, microwave powered lamps, xenon lamps, UV-LED lamps and laser beam sources such as excimer lasers and argon-ion lasers.

Where an e-beam is utilized to initiate the reaction, standard parameters for the operating device may be: an accelerating voltage of from 0.1 to 100 keV; a vacuum of from 10 to 10⁻³ Pa; an electron current of from 0.0001 to 1 ampere; and, power of from 0.1 watt to 1 kilowatt.

The amount of radiation necessary to sufficiently initiate the reaction will depend on a variety of factors including the angle of exposure to the radiation and the volume of the reaction mixture. Broadly, however, an applied dosage of from 5 to 5000 mJ/cm² may be cited as being typical: applied dosages of from 50 to 500 mJ/cm², such as from 50 to 200 mJ/cm² may be considered highly effective.

As would be recognized by the skilled artisan, photosensitizers can be incorporated into the reaction mixture to improve the efficiency with which a photoinitiator uses the energy delivered. The term *"photosensitizer"* is used in accordance with its standard meaning to represent any substance that either increases the rate of photoinitiated polymerization or shifts the wavelength at which polymerization occurs. Photosensitizers should be used in an amount of from 0 to 25 wt.%, based on the weight of said free radical photoinitiator.

In lieu of using a free radical photoinitiator, radical generating thermal initiators may be employed in this step. Organic peroxides represent one exemplary class thereof: such organic peroxides may be selected, for example, from: cyclic peroxides; diacyl peroxides; dialkyl peroxides; hydroperoxides; peroxycarbonates; peroxydicarbonates; peroxyesters; and, peroxyketals.

While certain peroxides - such as dialkyl peroxides - have been disclosed as useful initiators in *inter alia* US Patent No. 3,419,512 (Lees) and US Patent No. 3,479,246 (Stapleton) and indeed may have utility herein, hydroperoxides represent a preferred class of initiator. Further, whilst hydrogen peroxide itself may be used; the most desirable polymerization initiators are the organic hydroperoxides. For completeness, included within the definition of hydroperoxides are materials such as organic peroxides or organic peresters which decompose or hydrolyze to form organic hydroperoxides *in situ:* examples of such peroxides and peresters are cyclohexyl and hydroxycyclohexyl peroxide and t-butyl perbenzoate, respectively.

In an embodiment of the invention, the radical generating thermal initiator comprises or consists of at least one hydroperoxide compound represented by the formula:

R^{p}OOH

wherein: RP is an aliphatic or aromatic group containing up to 18 carbon atoms, and
preferably wherein: RP is a C₁-C₁₂ alkyl, C₆-C₁₈ aryl or C₇-C₁₈ aralkyl group.

As exemplary peroxide initiators, which may be used alone or in combination, there may be mentioned: cumene hydroperoxide (CHP); para-menthane hydroperoxide; t-butyl hydroperoxide (TBH); t-butyl perbenzoate; t-butyl peroxy pivalate; di-t-butyl peroxide; t-butyl peroxy acetate; t-butyl peroxy-2-hexanoate; t-amyl hydroperoxide; 1,2,3,4-tetramethylbutyl hydroperoxide; benzoyl peroxide; dibenzoyl peroxide; 1,3-bis(t-butylperoxyisopropyl) benzene; diacetyl peroxide; butyl 4,4-bis (t-butylperoxy) valerate; p-chlorobenzoyl peroxide; t-butyl cumyl peroxide; di-t-butyl peroxide; dicumyl peroxide; 2,5-dimethyl-2,5-di-t-butylperoxyhexane; 2,5-dimethyl-2,5-di-t-butyl-peroxyhex-3-yne; and, 4-methyl-2,2-di-t-butylperoxypentane.

Without intention to limit the present disclosure, a further exemplary class of radical generating thermal initiators suitable for use in this reaction step are azo polymerization initiators, selected for example from: azo nitriles; azo esters; azo amides; azo amidines; azo imidazoline; and, macro azo initiators.

As representative examples of suitable azo polymerization initiators there may be mentioned: 2,2'-azobis (2-methylbutyronitrile); 2,2'-azobis(isobutyronitrile) (AIBN); 2,2'-azobis(2,4-dimethylvaleronitrile); 2,2'-azobis(4-methoxy-2,4-dimethylvaleronitrile); 1,1'-azobis(cyclohexane-1-carbonitrile); 4,4'-azobis(4-cyanovaleric acid); dimethyl 2,2'-azobis(2-methylpropionate); 2,2'-azobis[2-methyl-N-(2-hydroxyethyl)propionamide]; 2,2'-azobis (N-butyl-2-methylpropionamide); 2,2'-azobis[2-(2-imidazolin-2-yl)propane]dihydrochloride; 2,2'-azobis[2-(2-imidazolin-2-yl)propane]; 2,2'-azobis(2-methylpropionamidine)dihydrochloride; 2,2'-azobis[N-(2-carboxyethyl)-2-methylpropionamidine]tetrahydrate; 4,4-azobis(4-cyanovaleric acid), polymer with alpha, omega-bis(3-aminopropyl)polydimethylsiloxane (VPS-1001, available from Wako Pure Chemical Industries, Ltd.); and, 4,4'-azobis(4-cyanopentanoicacic)-polyethyleneglycol polymer (VPE-0201, available from Wako Pure Chemical Industries, Ltd.).

Redox free radical initiators are a combination of an oxidizing agent and a reducing agent and may also have utility in in step b) based on the thiol-ene. Suitable oxidizing agents may be selected from the group consisting of cyclic peroxides, diacyl peroxides, dialkyl peroxides, hydroperoxides, peroxycarbonates, peroxydicarbonates, peroxyesters and peroxyketals. The corresponding reducing agent may be selected from the group consisting of: alkali metal sulfites; alkali metal hydrogensulfites; alkali metal metabisulfites; formaldehyde sulfoxylates; alkali metal salts of aliphatic sulfinic acids; alkali metal hydrogensulfides; salts of polyvalent metals, in particular Co(ll) salts and Fe(ll) salts such iron(II) sulfate, iron(II) ammonium sulfate or iron(II) phosphate; dihydroxymaleic acid; benzoin; ascorbic acid; and, reducing saccharides, such as sorbose, glucose, fructose and/or dihydroxyacetone.

Step b) may be performed at a temperature of from 5 to 150°C, with a temperature in the range from 20 to 100°C being more typical. The complete reaction may be accomplished in as little as one hour but, at lower reaction temperatures, as long as 24 hours may be required. Since the reaction is heterogeneous, agitation throughout the course of the reaction is important to maintain good physical contact between the reactants and to improve the reaction rate. Any means of agitation may be used.

The process pressure in step b) is not critical: as such, the reaction can be run at sub-atmospheric, atmospheric, or super-atmospheric pressures but pressures at or slightly above atmospheric pressure are preferred. Mention in this regard may be made of pressures of from 50 to 200 kPa, for example from 100 to 200 kPa.

The progress of the above reaction may be monitored by known techniques of which mention may be made of ¹H NMR, Fourier Transform Infrared Spectroscopy, Ultra Performance Liquid Chromatography (UPLC) or thin layer chromatography (TLC). Upon completion of the reaction, the unreacted thiol acid (IV) may be removed under reduced pressure to yield the crude thioester (V).

That crude product may be used in the subsequent step of the process or, alternatively, the relevant compound may be purified using methods known in the art, including but not limited to solvent extraction, filtration and chromatography.

### Step c) Hydrolysis

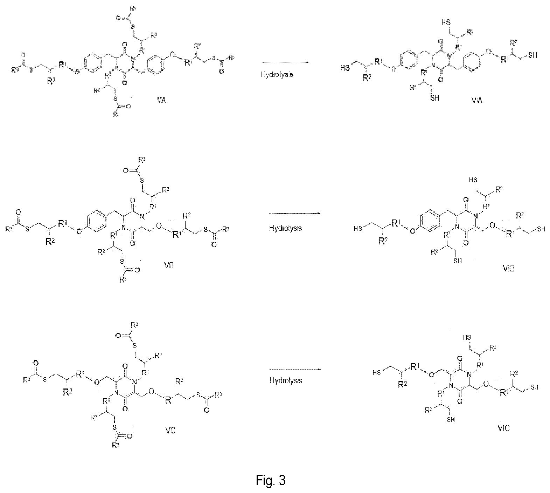

In this step of the process, depicted in Figure 3, the thioesters (V) are deprotected to yield the corresponding polythiol (VI). It is considered that this step may be performed using a reductive method wherein the thioester (IV) is reduced with LiAlH₄ with subsequent hydrolysis yielding the thiol (VI). However, in an important embodiment, this deprotection step is performed by acid-catalysed or base-catalysed hydrolysis.

In this embodiment, said acid or base catalyst should be present in the reaction mixture in an amount of from 0.05 to 0.25 moles per mole of said thioester (V). As regards, acid-based hydrolysis, common acids include H₂SO₄ and HCl. For base-catalysed hydrolysis, organic base catalysts are preferred of which non-limiting examples include: pyridine; dimethylaminopyridine (DMAP); proline; triazabicyclodecene (TBD); diazabicycloundecene (DBU); hexahydro methyl pyrimido pyridine (MTBD); diazabicyclononane (DBN); tetramethylguanidine (TMG); and, triethylenediamine (TED, *1,4-diazabicyclo[2.2.2]octane).*

The nucleophilic species (H) in the hydrolysis may be water. However, it is preferred that the nucleophile is a C₁-C₄ alkanol: a particular preference for the use of methanol (MeOH) may be noted.

Wherein a C₁-C₄ alkanol is used the reaction products will be the thiol (VI) and a C₁-C₄ alkyl ester. That aside, nucleophile (H) should be present in at least a stoichiometric amount to the number of moles of thioester groups of compound (IV). An excess of the nucleophile may serve to drive the reaction to completion.

Whilst not strictly required, the described reaction of this step c) may be carried out in the presence of an inert aprotic solvent. The use of ether solvents is preferred of which mention may be made of: tetrahydrofuran (THF); 2-methyltetrahydrofuran (2-MeTHF); 1,2-dimethoxyethane; and, 1,3-dioxolane.

The reaction need not be performed under reflux conditions but it is preferred to do so. Where reflux conditions are not used, the performance of the reaction at room temperature is not actually precluded but the use of elevated temperatures, for example at least 50°C or at least 75°C, can drive the reaction. Such elevated temperatures may indeed be required for acid-catalysed hydrolysis.

The process pressure is not critical: as such, the reaction can be run at sub-atmospheric, atmospheric, or super-atmospheric pressures but pressures at or slightly above atmospheric pressure are preferred. Mention in this regard may be made of pressures of from 50 to 200 kPa, for example from 100 to 200 kPa.

The progress of the above reaction may be monitored by known techniques of which mention may be made of ¹H NMR, Fourier Transform Infrared Spectroscopy, Ultra Performance Liquid Chromatography (UPLC) or thin layer chromatography (TLC). Upon completion of the reaction, the reaction mixture may be concentrated under evaporative conditions - to remove excess water, C₁-C₄ alkanol and / or solvent - to yield the crude polythiol (VI). That crude product may be purified using methods known in the art, including but not limited to solvent extraction, filtration and chromatography.

### SECOND PROCESS EMBODIMENT

In this process embodiment, the intermediate compound of Formula V is synthesized in a single step (α) from the compound of Formula I. This process does not proceed via an allyl functional intermediate.

### Step α)

In this step of the described process, depicted in Figure 4, there is provided the reaction of a compound of Formula I, in the presence of at least one base, with a thiolate ester having the general Formula VII: 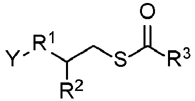 wherein:
Y is a halogen;
R¹ is a C₁-C₁₈ alkylene group, C₂-C₁₈ oxyalkylene group, C₂-C₁₈ thioalkylene group, C₆-C₁₈ arylene or C₇-C₁₈ aralkylene group;
R² is H or methyl; and,
R³ is C₁-C₆ alkyl, C₆-C₁₈ aryl, C₇-C₁₈ alkylaryl or C₇-C₁₈ aralkyl.

In most cases, the chloride (Y=Cl) is favoured because of lower cost, but the bromides and iodides may be more reactive, particularly in the case of high molecular weight compounds. In an initial statement of preference, it is preferred that: R¹ is a C₁-C₁₈ alkylene group, C₂-C₁₈ oxyalkylene group or C₂-C₁₈ thioalkylene group. In particular, it is preferred that: R¹ is an unsubstituted C₁-C₁₂ alkylene group; R² is H or Me; and, R³ is C₁-C₄ alkyl, C₆ aryl, C₇-C₁₈ alkylaryl or C₇-C₁₈ aralkyl. In important embodiments: R¹ is an unsubstituted C₁-C₄ alkylene group; R² is H or Me; and, R³ is a C₁-C₄ alkyl.

Exemplary thiolate esters in accordance with Formula VII include: S-(3-chloropropyl)ethanethioate; S-(3-bromopropyl)ethanethioate; and, S-(3-iodopropyl)ethanethioate.

It is intended that each hydroxyl group and each amine group in the compounds of Formula I be subjected to substitution and, as such, the thiolate ester compound (VII) should be present in at least a stoichiometric equivalent amount to the total number of moles of said hydroxyl and amine groups. The reaction mixture may, in particular, include said thiolate ester compound (VII) at a molar equivalency of from 1 to 2 relative to the total number of moles of the amine and hydroxyl groups of Formula I. Preferably, the thiolate ester compound (VII) is reacted at a molar equivalency of from 1.2 to 1.8 relative to the total number of moles of the amine and hydroxyl groups of Formula I.

The base which is present will react with the halogen acid or the C₁-C₄ alkyl carbonic acid liberated in the reaction and therefore should be present in at least the stoichiometric amount from that reaction. The reaction is operable in the presence of excess base however and therefore the total amount of base present in the reaction mixture may be from 1 to 5 molar equivalents to the amount of compound (I). It is preferred that the total amount of base is from 1.5 to 3 molar equivalents or from 1.5 to 2.5 molar equivalents to the amount of compound (I).

Without intention to limit the present invention, the base should desirably consist of at least one compound selected from alkali metals, alkaline earth metals, alkali metal (C₁-C₄)alkoxides, alkaline earth metal (C₁-C₄) alkoxides, alkali metal carbonates, alkaline earth metal carbonates, alkali metal hydroxides, alkaline earth metal hydroxides, alkali metal hydrides and alkaline earth metal hydrides. For substitution at the phenolic hydroxyl group, the use of at least one of potassium carbonate, sodium carbonate and calcium carbonate may be preferred: stronger bases may however be required for substitution at the constituent -NH groups. Given this, the present disclosure does not preclude the performance of the substitution in two sub-steps, wherein in a first sub-step substitution is effected at the phenolic hydroxyl groups under a first set of basic conditions and subsequently, in a second sub-step, substitution is effected at the remaining ring -NH groups of compound (I) under a second set of basic conditions. The separation and purification of the intermediate formed in the first sub-step, prior to the second sub-step, is not precluded in this embodiment.

In certain embodiments, the addition of a neutral alkali metal salt to the reaction mixture can have a positive effect on the yield of the thioester reaction product (V). Exemplary alkali metal salts include: sulfates; sulfites; sulfides; halides; nitrates; nitrites; cyanides; borates; phosphates; monohydrogen phosphates; dihydrogen phosphates; phosphites; acid phosphites; and, carboxylates, such as acetates, formates, propionates, oxalates, tartrates, succinates, maleates and adipates.

A small amount of the neutral alkali metal salt - for instance from 0.01 to 0.05 moles per mole of compound (I) - will have a yield-enhancing effect. The amount of said neutral salt should not however exceed the amount of basic catalyst included in the reaction mixture.

This step is still further performed in the presence of inert aprotic solvent. Examples of suitable aprotic solvents, which may be used alone or in combination, include but are not limited to: pentane; hexane; heptanes; cyclopentane; cyclohexane; cycloheptane; dimethylether; chloroform; dimethyl carbonate; ethylmethyl carbonate; diethyl carbonate; toluene; o-xylene; m-xylene; p-xylene; ethylbenzene; 2-propylbenzene (cumene); 2-isopropyltoluene (*o-cymol*); 3-isopropyltoluene (*m-cymol*); *4-*isopropyltoluene (*p-cymol*); 1,3,5-trimethylbenzene *(mesitylene);* acetonitrile; N,N-di(C₁-C₄)alkylacylamides, such as N,N-dimethylformamide (DMF) and N,N-dimethylacetamide (DMAc); hexamethylphosphoramide; N-methylpyrrolidone; pyridine; esters, such as (C₁-C₈)alkyl acetates, ethoxydiglycol acetate, dimethyl glutarate, dimethyl maleate, dipropyl oxalate, ethyl lactate, benzyl benzoate, butyloctyl benzoate and ethylhexyl benzoate; ketones, such as acetone, ethyl ketone, methyl ethyl ketone *(2-butanone)* and methyl isobutyl ketone; ethers, such as tetrahydrofuran (THF), 2-methyltetrahydrofuran (2-MeTHF) and 1,2-dimethoxyethane; 1,3-dioxolane; dimethylsulfoxide (DMSO); and, dichloromethane (DCM).

Whilst it is not critical, it is preferred that the reaction of this step be performed under anhydrous conditions. Where necessary, exposure to atmospheric moisture may be avoided by providing the reaction vessel with an inert, dry gaseous blanket. Whilst dry nitrogen, helium and argon may be used as blanket gases, precaution should be used when common nitrogen gases are used as a blanket, because such nitrogen may not be dry enough on account of its susceptibility to moisture entrainment; the nitrogen may require an additional drying step before use herein.

The reaction need not be performed under reflux conditions, but it is preferred to do so. Where reflux conditions are not used, the performance of the reaction at 0°C is not actually precluded but the use of elevated temperatures, for example at least 50°C or at least 75°C, can drive the reaction. For an exothermic reaction, some cooling might however be required as the reaction progresses.

The process pressure in step α) is not critical: as such, the reaction can be run at sub-atmospheric, atmospheric, or super-atmospheric pressures but pressures at or slightly above atmospheric pressure are preferred. Mention in this regard may be made of pressures of from 50 to 200 kPa, for example from 100 to 200 kPa.

The progress of the above reaction may be monitored by known techniques of which mention may be made of ¹H NMR, Fourier Transform Infrared Spectroscopy, Ultra Performance Liquid Chromatography (UPLC) or thin layer chromatography (TLC). Upon completion of the reaction, excess base present in the reaction mixture may be quenched using an appropriate acid, such as hydrochloric acid. Moreover, the inert aprotic solvent may be removed under reduced pressure.

The crude product may then be separated from the reaction obtained mixture and may be used *per* se in the subsequent step of the process. Alternatively, the relevant compound may be purified using methods known in the art, including but not limited to solvent extraction, filtration and chromatography.

### Step β) Hydrolysis

This step of the process, in which the thioesters (V) are deprotected to yield the corresponding polythiol (VI), is equivalent to step c) as described above and is depicted in Figure 3.

### EXEMPLARY COMPOUNDS

In accordance with exemplary but non-limiting embodiments of the present invention, there are provided compounds represented by the Formula VlAa and VICa below:

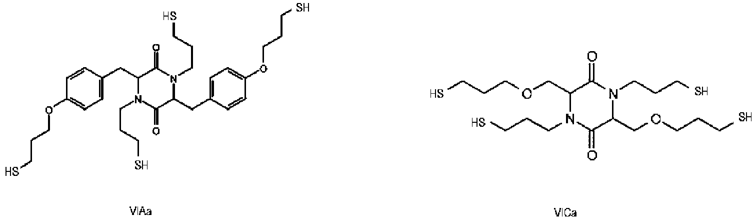

All stereoisomers of compounds VIAa and VICa are intended to be included in the scope of the present disclosure. However, particularly preferred stereoisomers are provided in Table 2 herein below:

**Table 2**

| Stereoisomer | Compound Name |
|---|---|
| 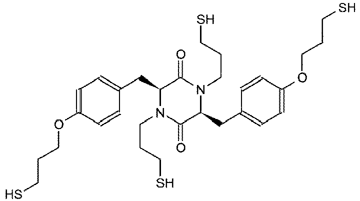 | (3S,6S)-3,6-bis[[4-(3-sulfanylpropoxy)phenyl]methyl]-1,4-bis(3-sulfanylpropyl)piperazine-2,5-dione |
| 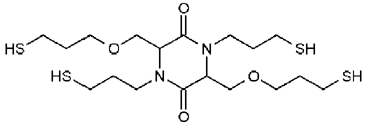 | 3,6-bis[(3-sulfanylpropoxy)methyl]-1,4-bis(3-sulfanylpropoxy)piperazine-2,5-dione, mixture of (3S,6S) and (3R,6S) isomers. |

In accordance with the above described reaction steps, these compounds may be formed from Compounds IA and IC:
i) in accordance with the first process embodiment, through using an allylation reagent (II) in which R¹=CH₂ and R²=H, in particular allyl bromide (X=Br) or ethyl prop-2-enyl carbonate (X is -OC=O)OEt); and, the thiol acid thioacetic acid (R³=Me); or,
ii) in accordance with the second process embodiment, through using a thiolate ester reagent (VII) in which Y=halogen, R¹=CH₂, R²=H and R³ is a C₁-C₄ alkyl.

### COMPOSITIONS CONTAINING THE THIOL COMPOUNDS OF THE PRESENT INVENTION

The polythiol compounds of the present disclosure are considered to be versatile and thereby have a plethora of uses. It is certainly anticipated that the thiol compounds *per se* may find utility as a curative, chain extender or otherwise reactive component of an one (1K) or two (2K) component curable composition. Such an one (1K) or two (2K) component curable composition may, in particular, be a coating, adhesive or sealant composition. It is also not precluded that the one (1K) or two (2K) composition may be a dual cure composition.

In an important embodiment of the disclosure, the curable composition comprises:
a) at least one polythiol as described hereinabove; and,
b) at least one thiol reactive compound, wherein the or each said thiol reactive compound has at least one functional group (F) selected from the group consisting of: epoxide groups; oxetane groups; cyclic carbonate groups; cyclic anhydride groups; 1-oxacycloalkan-2-one groups; ethylenically unsaturated groups; alkyne groups; and, isocyanate groups.

For surety, it is stated that a given thiol reactive compound b) may have more than one different functional group in its structure: it may, for instance, have an epoxide group and a (meth)acrylate group. Exemplary thiol reactive compounds b) include but are not limited to: epoxide compounds; ethylenically unsaturated compounds; epoxy (meth)acrylate compounds having at least one (meth)acrylate group and at least one epoxide group; and, polyisocyanates. As regards ethylenically unsaturated groups, note may be made of the instructive reference Hoyle et al. Thiol-Enes: Chemistry of the Past with Promise for the Future J. Polym. Sci. A Polym. Chem. 2004,42:5301 (2004).

No particular limitation is imposed on the number of functional groups (F) possessed by the thiol reactive compound(s): compounds having 1, 2, 3, 4, 5, 6, 7, 8, 9 or 10 functional groups may be used, for instance. Moreover, the thiol reactive compound(s) can be a low-molecular-weight substance - possessing a number average molecular weight (Mn) of less than 500 g/mol - or an oligomeric or polymeric substance that has a number average molecular weight (Mn) of at least 500 g/mol. And, of course, mixtures of thiol reactive compounds b) may be used.

The following examples are illustrative of the present invention and are not intended to limit the scope of the invention in any way.

### EXAMPLES

The following commercial compounds are employed in the Examples

| | |
|---|---|
| DCC: | Dicyclohexylcarbodiimide, coupling agent available from Sigma Aldrich |
| H-Tyr-OMe | L-Tyrosine methyl ester hydrochloride salt, available from Sigma Aldrich |
| Boc-Tyr-OH: | N-(tert-butoxycarbonyl)-L-tyrosine, available from Sigma Aldrich |
| DMF: | Dimethylformamide, available from Sigma Aldrich |
| MeCN: | Acetonitrile, available from Sigma Aldrich |
| L-Serine methyl ester hydrochloride: | Available from Akos Consulting and Solutions |

### Synthesis Example 1

### Cyclic Dimerization: Cyclo(L-Tyr-L-Tyr)

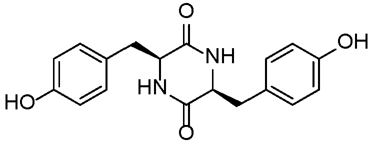

To a cooled solution (0°C) of H-Tyr-OMe (0.82 g, 3.6 mmol) and Boc-Tyr-OH (1.0 g, 3.6 mmol) in 4:1 MeCN: DMF (50 mL) was added triethylamine (0.48 mL, 3.6 mmol) and DCC (0.95 g, 3.6 mmol). The solution was then stirred for 3 hours at room temperature before cooling to -18 °C for 16 hours. The precipitated DCU was removed by filtration and the filtrate evaporated. The residue was partitioned between EtOAc (100 mL) and 1M HCl (100 mL). The aqueous phase was extracted with EtOAc (3 × 100 mL). The combined organic fractions were then washed with saturated NaHCOs (100 mL), brine (100 mL) and dried (MgSO₄). The solvent was removed under reduced pressure and the residue purified by flash chromatography (2:1, EtOAc: hexane) to afford Boc-Tyr-Tyr-OMe as a colourless solid (1.3 g, 3.0 mmol, 84% yield).

Boc-Tyr-Tyr-OMe 5 was dissolved in formic acid and stirred for 2 hours. The solvent was evaporated and trace formic acid was removed by azeotropic distillation with toluene (2 × 30 mL). The residue was dissolved in 4:1 sec-butanol: toluene (100 mL) and the solution was heated at reflux for 3 hours. The solvent was then removed under reduced pressure to afford the title compound as a colorless solid (880 mg, quantitative).

### Synthesis Example 2

### Cyclic Dimerization: Cyclo(Tyr-Tyr)

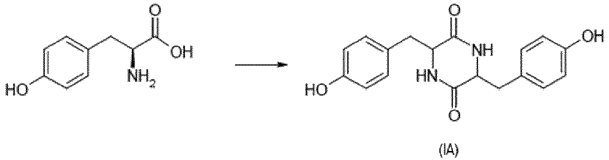

L-Tyrosine (300g, 1.66 mole, 1.0 equiv.) was suspended in ethylene glycol (1200mL) under N2 atmosphere and the mixture was heated to reflux for 18 hours. After cooling down to 23°C, water (1200mL) was added and the suspension was filtered over a sinter. The resulting solid was washed with water (3x600mL) and dried under reduced pressure (70°C). The orange solid was then suspended in ethanol (2500mL) and heated to reflux for 30 minutes. The suspension was filtered over a sinter and the solid washed with ethanol (500mL). Drying under reduced pressure (60°C) led to compound IA *(mix cis*/*trans 1:1.1; 175.2g; Purity:* >95%; *Yield:* 65%) as an off-white solid.

### Example 1

### Step a) i): Allylation Yielding 3,6-bis[(4-allyloxyphenyl)methyl]piperazine-2,5-dione

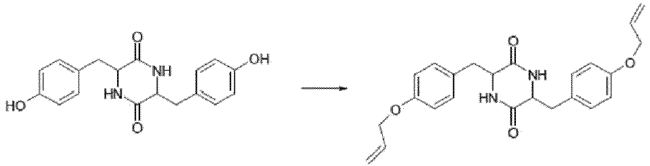

Compound IA (cis/trans 1.1:1, 130g, 398.3 mmol., 1.0 equiv.) and K₂CO₃ (220.2g, 1.59mol, 4.0 equiv.) were suspended in DMF (3790mL) under N₂ atmosphere. Allyl bromide (215.1g, 154.7mL, 1.59 mol., 4.0 equiv.) was added slowly over 10 minutes. The resulting mixture was heated at 55°C for 18 hours. The solvent was evaporated under reduced pressure (55°C). The residue was then suspended in water (6500mL) and the mixture was filtered over a sinter. The solid was washed with water (2x1000mL), suspended in toluene (1000mL) and evaporated under reduced pressure. The solid was suspended again two times in toluene (1000mL) and evaporated under reduced pressure. The solid was suspended a last time in toluene (1000mL), filtered over a sinter and washed with toluene (200mL). After drying under reduced pressure (60°C), the title compound (*mix cis*/*trans 1:1.1; 137.0g; Purity: 95%; Yield:* 85%) was obtained as an off-white solid.

### Step a) ii): Allylation to Yield 1,4-diallyl-3,6-bis[(4-allyloxyphenyl)methyl]piperazine-2,5-dione (IIIAa)

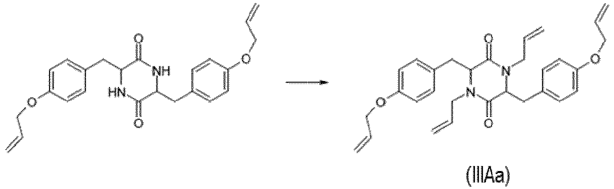

3,6-bis[(4-allyloxyphenyl)methyl]piperazine-2,5-dione (cis/trans 1.1:1, 137.0g, 337.0 mmol, 1.0 equiv.) as obtained in Step a)i) was suspended in DMF (2450mL) under N₂ atmosphere. The mixture was cooled to 0°C and NaH (60% in mineral oil, 27.0g, 674.0 mmol, 2.0 equiv.) was added. The suspension was allowed to stir 30 minutes at 0°C and then allyl bromide (91.0g, 65.5 mL, 674.0 mmol, 2.0 equiv.) was added dropwise over 20 minutes. The mixture was allowed to warm up to 23°C overnight. After cooling down to 5°C, water (60mL) was added over 5 minutes and the mixture was allowed to stir for 15 minutes. The solution was then evaporated under reduced pressure (50°C). To the residue was added water (3700mL) and the resulting was extracted with EtOAc (2x2500mL). Combined organic layers were washed with Brine (1250mL), dried over Na₂SO₄, filtered and evaporated under reduced pressure (50°C). The crude material was purified by column chromatography leading to the title compound IllAa [*trans (62.0g, purity: 95%, yield: 38%) and cis (87.4g, purity: 95%, yield:* 53%)] both as off-white solids.

### Step b): Thiol-ene Reaction to Yield [3-[(2S,5S)-2,5-bis[[4-(3-acetylsulfanylpropoxy)phenyl]methyl]-4-(3-acetylsulfanylpropyl)-3,6-dioxo-piperazin-1-yl]propyl] ethanethioate

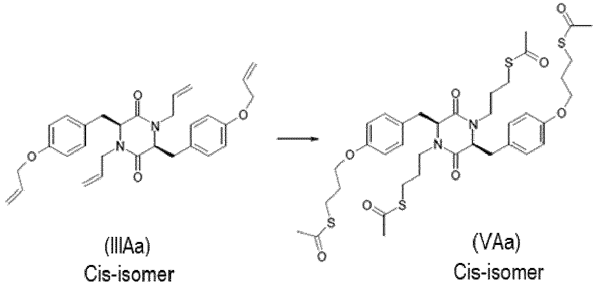

The cis-isomer of Compound IIIAa (85.0g, 174.7 mmol, 1.0 equiv.) was dissolved in dry THF (850mL) under N₂ atmosphere. Thioacetic acid (119.7g, 111.8mL, 1.57mol., 9.0 equiv.) was added over 5 minutes followed by AIBN (6.88g, 41.9 mmol, 0.24 equiv.). The mixture was heated at reflux for 20 hours. After cooling down to 23°C, the mixture was evaporated under reduced pressure (50°C). The residue was dissolved in 3 times in toluene (850mL) and evaporated under reduced pressure (50°C) again. The crude material was purified by column chromatography leading to compound VAa (*cis isomer; 116.8g; Purity: 95%; Yield:* 85%) as a pale yellow thick oil.

### Step c): Hydrolysis to Yield (3S,6S)-3,6-bis[[4-(3-sulfanylpropoxy)phenyl]methyl]-1,4-bis(3-sulfanylpropyl)-piperazine-2,5-dione

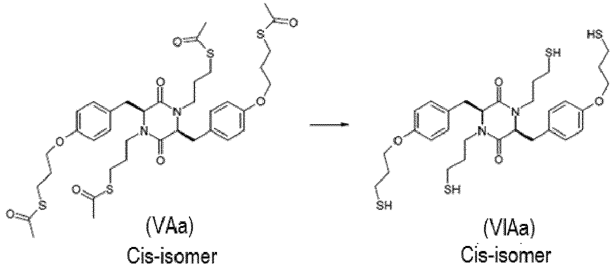

The cis-isomer of Compound VAa (90.0g, 113.8 mmol, 1.0 equiv.) as obtained in step b) was dissolved in a mixture of MeOH (930mL) and THF (93mL) under N₂ atmosphere. The solution was degassed by bubbling N₂ for 20 minutes. Concentrated HCl (49.3g, 41.8mL, 500.6 mmol, 4.4 equiv.) was then added and the solution was degassed again by bubbling N₂ for 20 minutes. The mixture was heated at 55°C for 20 hours before being cooled down to 23°C. Water (3000mL) was added and the mixture was extracted with DCM (3000+1000mL). Combined organic layers were successively washed with saturated NaHCOs (2000mL) and Brine (2000mL), dried over Na₂SO₄, filtered and evaporated under reduced pressure (40°C) leading to compound **VlAa** *(cis isomer; 71.2g; HPLC Purity: 96.8%; Yield: 100%)* as a pale yellow thick oil.

### Synthesis Example 3

### Cyclic Dimerization: (L-Ser-L-Ser)DKP

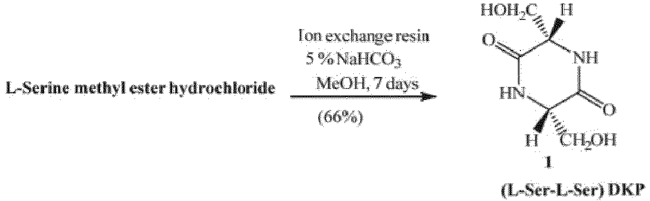

3,6-bis(hydroxymethyl)piperazine-2,5-dione was obtained by passing of a solution of L-Serine methyl ester hydrochloride in methanol through an ion exchange resin column that was pre-treated with 5% sodium bicarbonate. The desired dimer was obtained in 66% yield.

### Synthesis Example 4

### Cyclic Dimerization: 3,6-bis(hydroxymethyl)piperazine-2,5-dione (Ser-Ser)

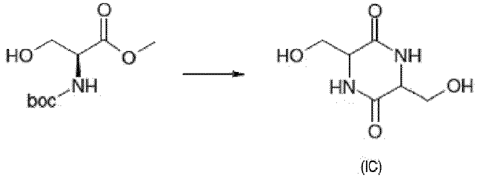

To a solution of N-Boc-L-serin methyl ester (414g, 1.89 mol., 1.0 equiv.) in dioxane (500mL), HCl in dioxane (4M, 1400mL, 3 equiv.) was added and the mixture was stirred at 25°C for 18 hours. The precipitated solid was filtered by suction, washed with dioxane (250mL) and dried under reduced pressure to obtain 217.46g of a white solid. This intermediate was dissolved in methanol (1000mL) and treated with NaOMe in MeOH (30%, 260mL, 1.4 mol.). The resulting solution was refluxed for 3 days. The precipitated solid was filtered by suction, washed with MeOH (2x 50mL) and dried under reduced pressure to obtain compound IC (65.74g; Purity: >95%; *Yield:* 40%; mixture of isomers 3:1) as a white powder.

### Example 2

### Step a): Allylation to Yield 1,4-diallyl-3,6-bis(allyloxymethyl)piperazine-2,5-dione

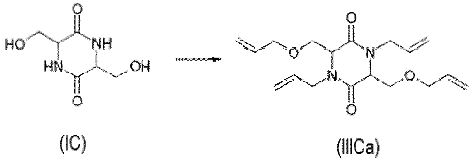

A solution of IC (50.00g, 0.29 mol., 1.0 equiv.) - as obtained in Synthesis Example 4 - in dry THF (900mL) was treated with allyl ethyl carbonate (186.8g, 1.44 mol., 5 equiv.) and the mixture was heated to reflux for 20 hours. The precipitated solid was filtered by suction and washed with THF (2x 100mL). The filtrate was concentrated under reduced pressure and the resulting oil was subjected to column chromatography (*silica gel; mobile phase of ethyl acetate*/*petrol ether, 1:1*) to obtain compound IIICa (*53.64g; Purity: >95%; Yield:* 56%; *mixture* of isomers, 3:1) as brown oil.

### Steps b) and c): Thiol-ene reaction followed by hydrolysis to yield 3,6-bis(3-sulfanylpropoxymethyl)-1,4-bis(3-sulfanylpropyl)piperazine-2,5-dione

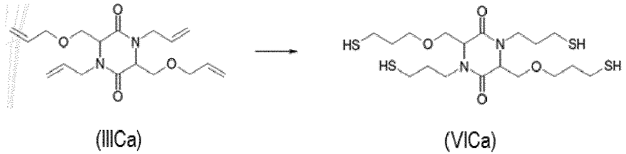

Compound IIICa (53.0g, 159mmol, 1.0equiv.) was dissolved in dry THF (800mL) under N₂ atmosphere. Thioacetic acid (108.6g, 101.5mL, 1.43 mol., 9.0 equiv.) was added over 5 minutes followed by AIBN (6.25g, 38 mmol., 0.24 equiv.). The mixture was heated at reflux for 20 hours. After cooling down to 23°C, the mixture was evaporated under reduced pressure (50°C). The residue was dissolved in 2 times in toluene (800mL) and evaporated under reduced pressure (50°C) again. The crude material was purified by column chromatography (*silica gel; mobile phase of ethyl acetate*/*petrol ether,* 2:1) to obtain the intermediate S-[3-[2,5-bis(3-acetylsulfanylpropoxymethyl)-4-(3-acetylsulfanylpropyl)-3,6-dioxo-piperazin-1-yl]propyl] ethanethioate (*91.23g; Purity: 95%; Yield:* 90%) as a pale yellow thick oil.

The intermediate (91.0g, 142.4 mmol.) was dissolved in a mixture of MeOH (1000mL) and THF (100mL) under N₂ atmosphere. The solution was degassed by bubbling N₂ for 20min. Concentrated HCl (61.9g, 52.5mL, 628 mmol., 4.4 equiv.) was then added and the solution was degassed again by bubbling N₂ for 20min. The mixture was heated at 55°C for 20h before being cooled down to 23°C. Water (3000mL) was added and the mixture was extracted with DCM (3000+1000mL). Combined organic layers were successively washed with saturated NaHCOs (2000mL) and brine (2000mL), dried over Na₂SO₄, filtered and evaporated under reduced pressure (40°C) leading to the title compound VICa (*66.05g; HPLC Purity: 96.4%; Yield: 89%; mixture of isomers* 5.5:1) as a pale yellow thick oil.

## Claims

1. A polythiol compound having the general Formula VIA, VIB or VIC: 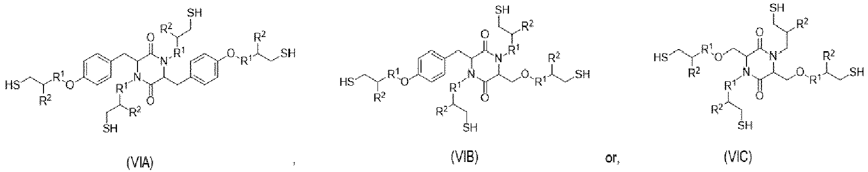 wherein:
R¹ is a C₁-C₁₈ alkylene group, C₂-C₁₈ oxyalkylene group, C₂-C₁₈thioalkylene
group, C₆-C₁₈ arylene or C₇-C₁₈ aralkylene group, wherein alkylene groups, oxyalkylene group and thioalkylene group may be unsubstituted or may be substituted with one or more halogen or hydroxyl; and arylene groups may be unsubstituted or may be substituted with one or more halogen; and,
R² is H or methyl.

2. The compound according to claim 1, wherein:
R¹ is an unsubstituted C₁-C₁₂ alkylene group, preferably an unsubstituted C₁-C₄ alkylene group; and,
R² is H or Me.

3. The compound according to claim 1 or claim 2 which is selected from:

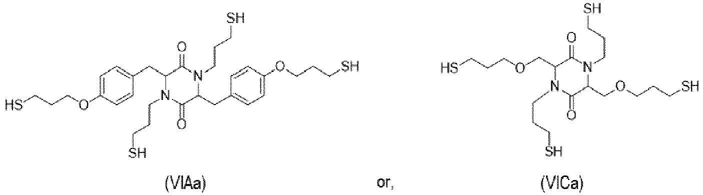

4. A process for preparing a compound as defined in any one of claims 1 to 3, said process comprising providing a compound having the general Formula IA, IB or IC: 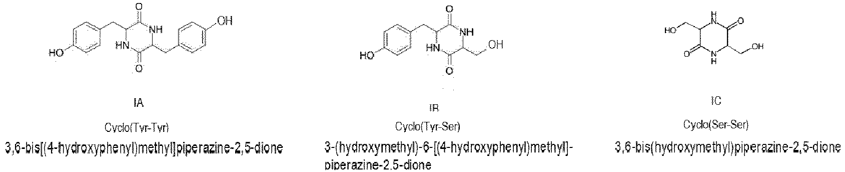 and further comprising the steps of
b) reacting, in the presence of at least one base, said compound of general Formula IA, IB or IC with a primary allyl compound (II) having the general formula:
X-(R¹)-C(R²)=CH₂ (II)
wherein:
X is a halogen or an -OC(=O)OR° group;
R° is a C₁-C₄ alkyl group;
R¹ is a C₁-C₁₈ alkylene group, C₂-C₁₈ oxyalkylene group, C₂-C₁₈
thioalkylene group, C₆-C₁₈ arylene or C₇-C₁₈ aralkylene group, wherein alkylene groups, oxyalkylene group and thioalkylene group may be unsubstituted or may be substituted with one or more halogen or hydroxyl; and arylene groups may be unsubstituted or may be substituted with one or more halogen; and,
R² is H or methyl,
to form, respectively, a compound of Formula IIIA, IIIB or IIIC: 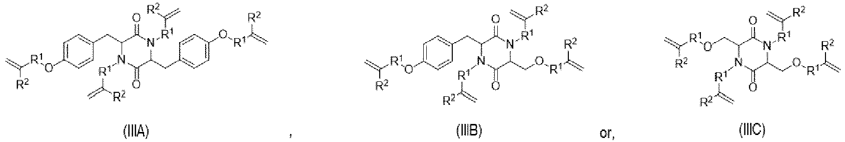
b) reacting said compound of Formula IIIA, IIIB or IIIC with a thiol acid (IV) having the general formula
R³-C(=O)-SH (IV)
wherein: R³ is C₁-C₆ alkyl, C₆-C₁₈ aryl, C₇-C₁₈ alkylaryl or C₇-C₁₈ aralkyl, wherein alkyl and aryl groups may be unsubstituted or may be substituted with one or more halogen,
to form, respectively, a thioester compound of Formula VA, VB or VC: 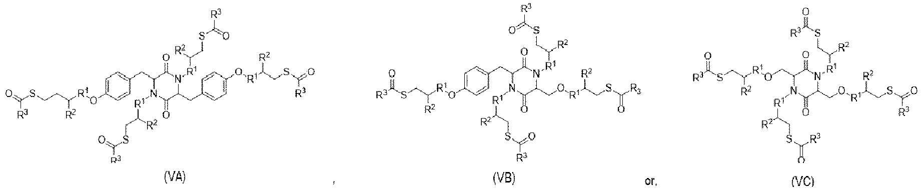 and,
c) deprotecting said thioester compound of Formula VA, VB or VC to form, respectively, said polythiol compound of Formula VIA, VIB or VIC.

5. The process according to claim 4, wherein said primary allyl compound (II) is **characterized in that**:
R° is a C₁-C₂ alkyl;
R¹ is an unsubstituted C₁-C₁₂ alkylene group, preferably an unsubstituted C₁-C₄ alkylene group; and,
R² is H or Me.

6. The process according to claim 5, wherein said primary allyl compound (II) is selected from the group consisting of: 3-chloro-1-propene, 3-bromoprop-1-ene; 3-iodoprop-1-ene; methyl prop-2-enyl carbonate; and, ethyl prop-2-enyl carbonate.

7. The process according to any one of claims 4 to 6, wherein said primary allyl compound (II) is reacted at a molar equivalency of from 1 to 2 relative to the total number of moles of the amine and hydroxyl groups of Formula IA, IB or IC.

8. The process according to any one of claims 4 to 7, wherein said at least one base is present in step a) in an amount of from 1 to 5 molar equivalents relative to the reactant compound IA, IB or IC.

9. The process according to any one of claims 4 to 8, wherein said at least one base is selected from the group consisting of potassium carbonate, sodium carbonate and calcium carbonate.

10. The process according to any one of claims 4 to 9, wherein said thiol acid (IV) is **characterized in that**:
R³ is C₁-C₄ alkyl, C₆ aryl, C₇-C₁₈ alkylaryl or C₇-C₁₈ aralkyl; and,
wherein said thiol acid (IV) is preferably selected from the group consisting of thioacetic acid, thiopropionic acid; thiobutyric acid, thioisobutyric acid, thiobenzoic acid, 2-methyl-thiobenzoic acid, 3-methyl-thiobenzoic acid, 4-methyl-thiobenzoic acid, 2,4-dimethyl-thiobenzoic acid and 3,5-dimethyl-thiobenzoic acid.

11. The process according to any one of claims 4 to 10, wherein said thiol acid (IV) is reacted at a molar equivalency of from 1 to 2 relative to the allyl groups of said compound of Formula IIIA, IIIB or IIIC.

12. A process for preparing a compound as defined in any one of claims 1 to 3, said process comprising providing a compound having the general Formula IA, IB or IC: 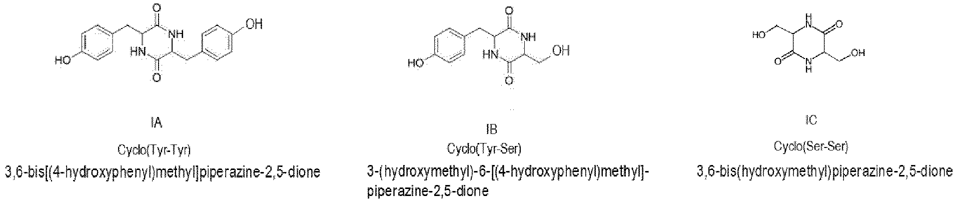 and further comprising the steps of:
α) reacting, in the presence of at least one base, said compound of general Formula IA, IB or IC with a thiolate ester compound having general Formula VII: 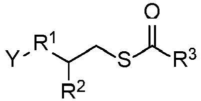 wherein:
Y denotes a halogen;
R¹ is a C₁-C₁₈ alkylene group, C₂-C₁₈ oxyalkylene group, C₂-C₁₈
thioalkylene group, C₆-C₁₈ arylene or C₇-C₁₈ aralkylene group,wherein alkylene groups, oxyalkylene group and thioalkylene group may be unsubstituted or may be substituted with one or more halogen or hydroxyl; and arylene groups may be unsubstituted or may be substituted with one or more halogen;
R² is H or methyl; and,
R³ is C₁-C₆ alkyl, C₆-C₁₈ aryl, C₇-C₁₈ alkylaryl or C₇-C₁₈ aralkyl, wherein alkyl and aryl groups may be unsubstituted or may be substituted with one or more halogen,
to form, respectively, a thioester compound of Formula VA, VB or VC: 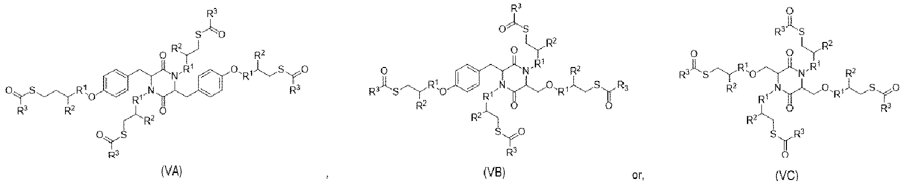 and,
β) deprotecting said thioester compound of Formula VA, VB or VC to form, respectively, said polythiol compound of Formula VIA, VIB or VIC.

13. The process according to any one of claims 4 to 12, wherein said deprotection step (c), β)) is performed by acid-catalysed or base-catalysed hydrolysis.

14. The process according to claim 13, wherein said acid or base catalyst is present in said deprotection step (c), β)) in an amount of from 0.05 to 0.25 moles per mole of said thioester of Formula VA, VB or VC.

15. The process according to claim 13 or claim 14, wherein said deprotection step (c), β)) is performed by base-catalysed hydrolysis and further wherein said base is selected from the group consisting of: pyridine; dimethylaminopyridine (DMAP); proline; triazabicyclodecene (TBD); diazabicycloundecene (DBU); hexahydro methyl pyrimido pyridine (MTBD); diazabicyclononane (DBN); tetramethylguanidine (TMG); and, triethylenediamine (TED, *1,4-diazabicyclo[2.2.2]octane).*

16. The process according to any one of claims 4 to 15, wherein said
said compound of Formula IA is provided by the homo-cyclization of tyrosine;
said compound of Formula IB is provided by the hetero-cyclization of tyrosine and serine; and,
said compound of Formula IC is provided by the homo-cyclization of serine.

17. Use of the polythiol compound as defined in any one of claims 1 to 3 as a reactive component of an one (1K) or two (2K) component curable composition.

18. A curable composition comprising:
c) at least one polythiol as defined in any one of claims 1 to 3; and,
d) at least one thiol reactive compound, wherein the or each said thiol reactive compound has at least one functional group (F) selected from the group consisting of: epoxide groups; oxetane groups; cyclic carbonate groups; cyclic anhydride groups; 1-oxacycloalkan-2-one groups; ethylenically unsaturated groups; alkyne groups; and, isocyanate groups, wherein said thiol reactive compound b) is preferably selected from the group consisting of include but are not limited to: epoxide compounds; ethylenically unsaturated compounds; epoxy (meth)acrylate compounds having at least one (meth)acrylate group and at least one epoxide group; and, polyisocyanates.

## Patentansprüche

1. Polythiolverbindung, die die allgemeine Formel VIA, VIB oder VIC aufweist: 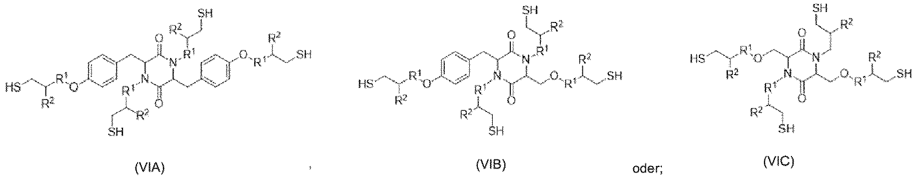
wobei: R¹ eine C₁-C₁₈-Alkylengruppe, C₂-C₁₈-Oxyalkylengruppe, C₂-C₁₈-Thioalkylengruppe, C₆-C₁₈-Arylen- oder C₇-C₁₈-Aralkylengruppe ist, wobei die Alkylengruppen, die Oxyalkylengruppe und die Thioalkylengruppe unsubstituiert sein können oder mit einem oder mehreren Halogenen oder Hydroxylen substituiert sein können; und Arylengruppen unsubstituiert sein können oder mit einem oder mehreren Halogenen substituiert sein können; ; und
R² H oder Methyl ist.

2. Verbindung nach Anspruch 1, wobei:
R¹ eine unsubstituierte C₁-C₁₂-Alkylengruppe, vorzugsweise eine unsubstituierte C₁-C₄-Alkylengruppe ist; und
R² H oder Me ist.

3. Verbindung nach Anspruch 1 oder 2, die ausgewählt ist aus:

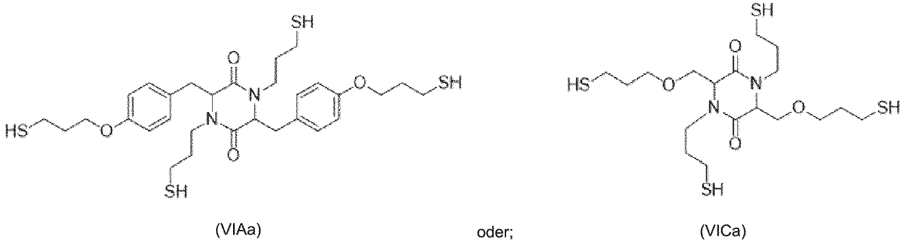

4. Verfahren für eine Herstellung einer Verbindung wie in einem der Ansprüche 1 bis 3 definiert, das Verfahren umfassend ein Bereitstellen einer Verbindung, die die allgemeine Formel IA, IB oder IC aufweist: 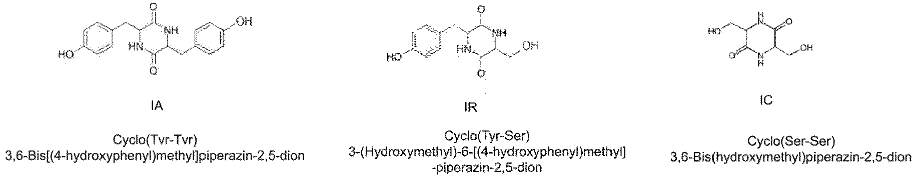 und ferner umfassend die Schritte
b) Umsetzen, in der Gegenwart mindestens einer Base, der Verbindung der allgemeinen Formel IA, IB oder IC mit einer primären Allylverbindung (II), die die allgemeine Formel aufweist:
X-(R¹)-C(R²)=CH₂ (II)
wobei:
X ein Halogen oder eine -OC(=O)OR°-Gruppe ist;
R° eine C₁-C₄-Alkylgruppe ist;
R¹ eine C₁-C₁₈-Alkylengruppe, C₂-C₁₈-Oxyalkylengruppe, C₂-C₁₈-Thioalkylengruppe, C₈-C₁₈-Arylen- oder C₇-C₁₈-Aralkylengruppe ist, wobei die Alkylengruppen, die Oxyalkylengruppe und die Thioalkylengruppe unsubstituiert sein können oder mit einem oder mehreren Halogenen oder Hydroxylen substituiert sein können; und Arylengruppen unsubstituiert sein können oder mit einem oder mehreren Halogenen substituiert sein können; und
R² H oder Methyl ist,
um, jeweils, eine Verbindung von Formel IIIA, IIIB oder IIIC auszubilden: 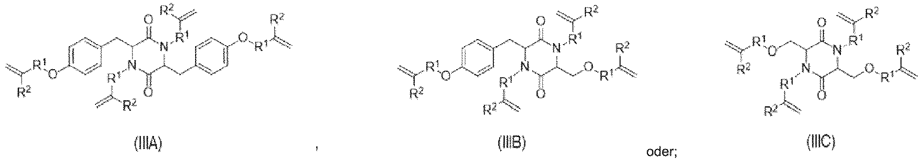
b) Umsetzen der Verbindung von Formel IIIA, IIIB oder IIIC mit einer Thiolsäure (IV), die die allgemeine Formel aufweist
R³-C(=O)-SH (IV)
wobei: R³ C₁-C₆-Alkyl, C₆-C₁₈-Aryl, C₇-C₁₈-Alkylaryl oder C₇-C₁₈-Aralkyl ist, wobei die Alkyl- und Arylgruppen unsubstituiert sein können oder mit einem oder mehreren Halogenen substituiert sein können, um, jeweils, eine Thioesterverbindung von Formel VA, VB oder VC auszubilden: 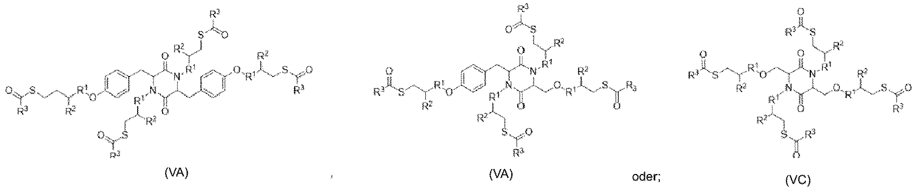 und
c) Deprotektieren der Thioesterverbindung von Formel VA, VB oder VC, um, jeweils, die Polythiolverbindung von Formel VIA, VIB oder VIC auszubilden.

5. Verfahren nach Anspruch 4, wobei die primäre Allylverbindung (II) **dadurch gekennzeichnet ist, dass:**
R° C₁-C₂-Alkyl ist;
R¹ eine unsubstituierte C₁-C₁₂-Alkylengruppe, vorzugsweise eine unsubstituierte C₁-C₄-Alkylengruppe ist; und
R² H oder Me ist.

6. Verfahren nach Anspruch 5, wobei die primäre Allylverbindung (II) aus der Gruppe ausgewählt ist, bestehend aus: 3-Chlor-1-propen, 3-Bromprop-1-en; 3-lodprop-1-en; Methylprop-2-enylcarbonat; und Ethylprop-2-enylcarbonat.

7. Verfahren nach einem der Ansprüche 4 bis 6, wobei die primäre Allylverbindung (II) bei einem Moläquivalent von 1 bis 2 relativ zu der Gesamtzahl von Mol der Amin- und Hydroxylgruppen von Formel IA, IB oder IC umgesetzt wird.

8. Verfahren nach einem der Ansprüche 4 bis 7, wobei die mindestens eine Base in Schritt a) in einer Menge von 1 bis 5 Moläquivalenten relativ zu der Reaktantenverbindung IA, IB oder IC vorhanden ist.

9. Verfahren nach einem der Ansprüche 4 bis 8, wobei die mindestens eine Base aus der Gruppe ausgewählt wird, bestehend aus Kaliumcarbonat, Natriumcarbonat und Calciumcarbonat.

10. Verfahren nach einem der Ansprüche 4 bis 9, wobei die Thiolsäure (IV) **dadurch gekennzeichnet ist, dass:**
R³ C₁-C₄-Alkyl, C₆-Aryl, C₇-C₁₈-Alkylaryl oder C₇-C₁₈-Aralkyl ist; und
wobei die Thiolsäure (IV) vorzugsweise aus der Gruppe ausgewählt ist, bestehend aus Thioessigsäure und Thiopropionsäure; Thiobuttersäure, Thioisobuttersäure, Thiobenzoesäure, 2-Methyl-thiobenzoesäure, 3-Methyl-thiobenzoesäure, 4-Methyl-thiobenzoesäure, 2,4-Dimethyl-thiobenzoesäure und 3,5-Dimethyl-thiobenzoesäure.

11. Verfahren nach einem der Ansprüche 4 bis 10, wobei die Thiolsäure (IV) in einem Moläquivalent von 1 bis 2 relativ zu den Allylgruppen der Verbindung von Formel IIIA, IIIB oder IIIC umgesetzt wird.

12. Verfahren für die Herstellung einer Verbindung wie in einem der Ansprüche 1 bis 3 definiert, das Verfahren umfassend ein Bereitstellen einer Verbindung, die die allgemeine Formel IA, IB oder IC aufweist: 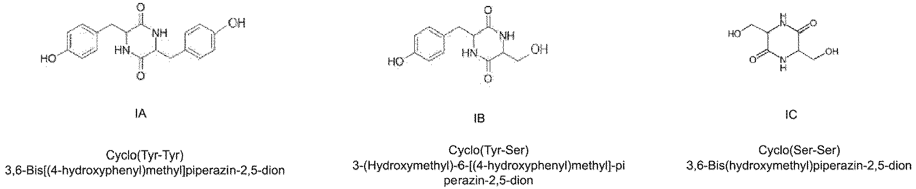 und ferner umfassend die Schritte:
α) Umsetzen, in der Gegenwart mindestens einer Base, der Verbindung der allgemeinen Formel IA, IB oder IC mit einer Thiolatesterverbindung, die die allgemeine Formel VII aufweist: 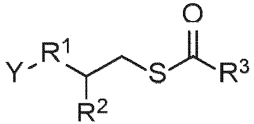
wobei: Y ein Halogen bezeichnet;
R¹ eine C₁-C₁₈-Alkylengruppe, C₂-C₁₈-Oxyalkylengruppe, C₂-C₁₈-Thioalkylengruppe, C₆-C₁₈-Arylen- oder C₇-C₁₈-Aralkylengruppe ist, wobei die Alkylengruppen, die Oxyalkylengruppe und die Thioalkylengruppe unsubstituiert sein können oder mit einem oder mehreren Halogenen oder Hydroxylen substituiert sein können; und Arylengruppen unsubstituiert sein können oder mit einem oder mehreren Halogenen substituiert sein können;
R² H oder Methyl ist; und
R³ C₁-C₆-Alkyl, C₆-C₁₈-Aryl, C₇-C₁₈-Alkylaryl oder C₇-C₁₈-Aralkyl ist, wobei die Alkyl- und Arylgruppen unsubstituiert sein können oder mit einem oder mehreren Halogenen substituiert sein können,
um, jeweils, eine Thioesterverbindung von Formel VA, VB oder VC auszubilden: 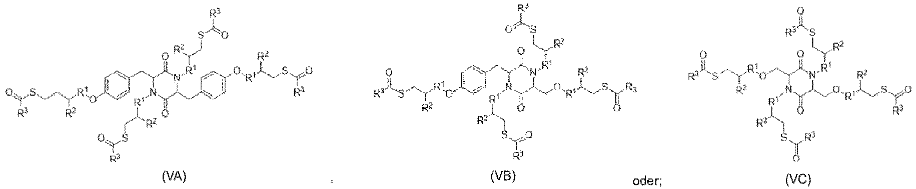 und
β) Deptrotektieren der Thioesterverbindung von Formel VA, VB oder VC, um, jeweils, die Polythiolverbindung von Formel VIA, VIB oder VIC auszubilden.

13. Verfahren nach einem der Ansprüche 4 bis 12, wobei der Deprotektierungsschritt (c), β)) durch eine säurekatalysierte oder basenkatalysierte Hydrolyse durchgeführt wird.

14. Verfahren nach Anspruch 13, wobei der Säure- oder Basenkatalysator in dem Deprotektierungsschritt (c), β)) in einer Menge von 0,05 bis 0,25 Mol pro Mol des Thioesters von Formel VA, VB oder VC vorhanden ist.

15. Verfahren nach Anspruch 13 oder 14, wobei der Deprotektierungsschritt (c), β)) durch basenkatalysierte Hydrolyse durchgeführt wird und ferner wobei die Base aus der Gruppe ausgewählt ist, bestehend aus Pyridin; Dimethylaminopyridin (DMAP); Prolin; Triazabicyclodecen (TBD); Diazabicycloundecen (DBU); Hexahydromethylpyrimidopyridin (MTBD); Diazabicyclononan (DBN); Tetramethylguanidin (TMG); und Triethylendiamin (TED, *1,4-Diazabicyclo[2.2.2]octan).*

16. Verfahren nach einem der Ansprüche 4 bis 15, wobei die
die Verbindung von Formel IA durch die Homocyclisierung von Tyrosin bereitgestellt wird;
die Verbindung von Formel IB durch die Heterocyclisierung von Tyrosin und Serin bereitgestellt wird; und
die Verbindung von Formel IC durch die Homocyclisierung von Serin bereitgestellt wird.

17. Verwendung der Polythiolverbindung wie nach einem der Ansprüche 1 bis 3 definiert als eine reaktive Komponente einer aushärtbaren Ein- (1K) oder Zwei-(2K)komponentenzusammensetzung.

18. Härtbare Zusammensetzung, umfassend:
c) mindestens ein Polythiol wie nach einem der Ansprüche 1 bis 3 definiert; und
d) mindestens eine Thiol-reaktive Verbindung, wobei die oder jede der Thiolreaktiven Verbindungen mindestens eine funktionelle Gruppe (F) aufweist, die aus der Gruppe ausgewählt ist, bestehend aus: Epoxidgruppen; Oxetangruppen; zyklischen Carbonatgruppen; zyklischen Anhydridgruppen; 1-Oxacycloalkan-2-on-Gruppen; ethylenisch ungesättigten Gruppen; Alkingruppen; und Isocyanatgruppen, wobei die Thiol-reaktive Verbindung b) vorzugsweise aus der Gruppe ausgewählt ist, bestehend aus, einschließlich, jedoch nicht beschränkt auf: Epoxidverbindungen; ethylenisch ungesättigten Verbindungen; Epoxid(meth)acrylatverbindungen, die mindestens eine (Meth)acrylatgruppe und mindestens eine Epoxidgruppe aufweisen; und Polyisocyanaten.

## Revendications

1. Composé polythiol ayant la Formule générale VIA, VIB ou VIC : 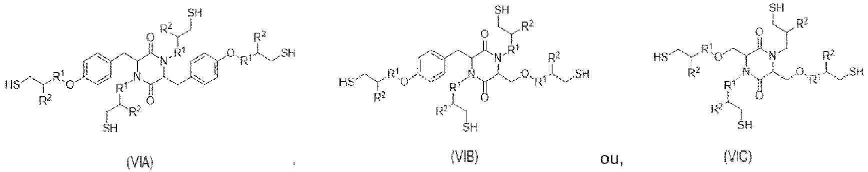 dans lequel :
R¹ est un groupe alkylène en C₁ à C₁₈, un groupe oxyalkylène en C₂ à C₁₈, un groupe thioalkylène en C₂ à C₁₈, un groupe arylène en C₆ à C₁₈ ou aralkylène en C₇ à C₁₈, dans lequel les groupes alkylène, le groupe oxyalkylène et le groupe thioalkylène peuvent être non substitués ou peuvent être substitués avec un halogène ou hydroxyle ou plus ; et les groupes arylène peuvent être non substitués ou peuvent être substitués avec un halogène ou plus ; ; et,
R² est H ou méthyle.

2. Composé selon la revendication 1, dans lequel :
R¹ est un groupe alkylène en C₁ à C₁₂ non substitué, de préférence un groupe alkylène en C₁ à C₄ non substitué ; et,
R² est H ou Me.

3. Composé selon la revendication 1 ou la revendication 2 qui est choisi parmi :

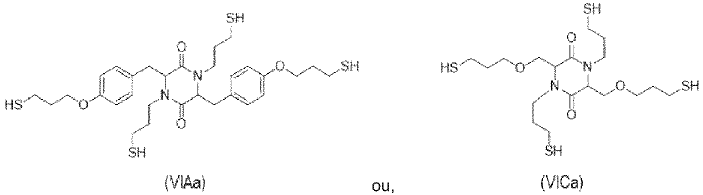

4. Procédé permettant de préparer un composé selon l'une quelconque des revendications 1 à 3, ledit procédé comprenant la fourniture d'un composé ayant la Formule générale IA, IB ou IC : 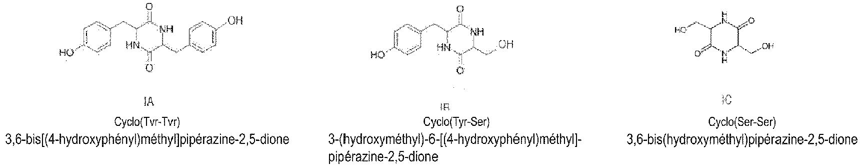 et comprenant en outre les étapes consistant à
b) faire réagir, en présence d'au moins une base, ledit composé de Formule générale IA, IB ou IC avec un composé allyle primaire (II) ayant la formule générale :
X-(R¹)-C(R²)=CH₂ (II)
dans lequel :
X est un halogène ou un groupe -OC(=O)OR° ;
R° est un groupe alkyle en C₁ à C₄ ;
R¹ est un groupe alkylène en C₁ à C₁₈, un groupe oxyalkylène en C₂ à C₁₈, un groupe thioalkylène en C₂ à C₁₈, un groupe arylène en C₈ à C₁₈ ou aralkylène en C₇ à C₁₈, dans lequel les groupes alkylène, le groupe oxyalkylène et le groupe thioalkylène peuvent être non substitués ou peuvent être substitués avec un halogène ou hydroxyle ou plus ; et les groupes arylène peuvent être non substitués ou peuvent être substitués avec un halogène ou plus ; et
R² est H ou méthyle,
pour former, respectivement, un composé de Formule IIIA, IIIB ou IIIC : 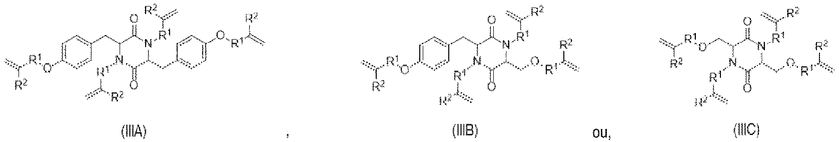
b) faire réagir ledit composé de Formule IIIA, IIIB ou IIIC avec un acide thiol (IV) ayant la formule générale
R³-C(=O)-SH (IV)
dans lequel : R³ est alkyle en C₁ à C₆, aryle en C₆ à C₁₈, alkylaryle en C₇ à C₁₈ ou aralkyle en C₇ à C₁₈, dans lequel les groupes alkyle et aryle peuvent être non substitués ou peuvent être substitués avec un halogène ou plus, pour former, respectivement, un composé thioester de Formule VA, VB ou VC : 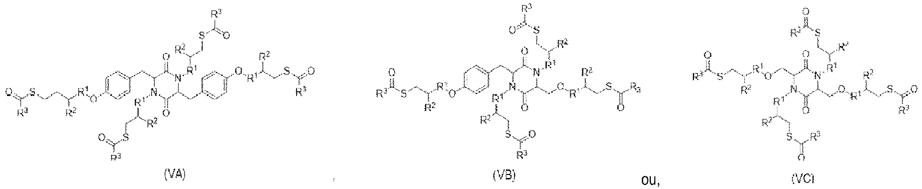 et,
c) déprotéger ledit composé thioester de Formule VA, VB ou VC pour former, respectivement, ledit composé polythiol de Formule VIA, VIB ou VIC.

5. Procédé selon la revendication 4, dans lequel ledit composé allyle primaire (II) est **caractérisé en ce que :**
R° est un alkyle en C₁ à C₂ ;
R¹ est un groupe alkylène en C₁ à C₁₂ non substitué, de préférence un groupe alkylène en C₁ à C₄ non substitué ; et,
R² est H ou Me.

6. Procédé selon la revendication 5, dans lequel ledit composé allyle primaire (II) est choisi dans le groupe constitué par : 3-chloro-1-propène, 3-bromoprop-1-ène ; 3-iodoprop-1-ène ; carbonate de méthyl-prop-2-ényle ; et, carbonate d'éthyl-prop-2-ényle.

7. Procédé selon l'une quelconque des revendications 4 à 6, dans lequel ledit composé allyle primaire (II) est mis en réaction à une équivalence molaire allant de 1 à 2 par rapport au nombre total de moles des groupes amine et hydroxyle de Formule IA, IB ou IC.

8. Procédé selon l'une quelconque des revendications 4 à 7, dans lequel ladite au moins une base est présente à l'étape a) en une quantité allant de 1 à 5 équivalents molaires par rapport au composé réactif IA, IB ou IC.

9. Procédé selon l'une quelconque des revendications 4 à 8, dans lequel ladite au moins une base est choisie dans le groupe constitué par carbonate de potassium, carbonate de sodium et carbonate de calcium.

10. Procédé selon l'une quelconque des revendications 4 à 9, dans lequel ledit acide thiol (IV) est **caractérisé en ce que :**
R³ est alkyle en C₁ à C₄, aryle en C₆, alkylaryle en C₇ à C₁₈ ou aralkyle en C₇ à C₁₈ ; et,
dans lequel ledit acide thiol (IV) est de préférence choisi dans le groupe constitué par acide thioacétique, acide thiopropionique ; acide thiobutyrique, acide thio-isobutyrique, acide thiobenzoïque, acide 2-méthyl-thiobenzoïque, acide 3-méthyl-thiobenzoïque, acide 4-méthyl-thiobenzoïque, acide 2,4-diméthyl-thiobenzoïque et acide 3,5-diméthyl-thiobenzoïque.

11. Procédé selon l'une quelconque des revendications 4 à 10, dans lequel ledit acide thiol (IV) est mis en réaction à une équivalence molaire allant de 1 à 2 par rapport aux groupes allyle dudit composé de Formule IIIA, IIIB ou IIIC.

12. Procédé permettant de préparer un composé selon l'une quelconque des revendications 1 à 3, ledit procédé comprenant la fourniture d'un composé ayant la Formule générale IA, IB ou IC : 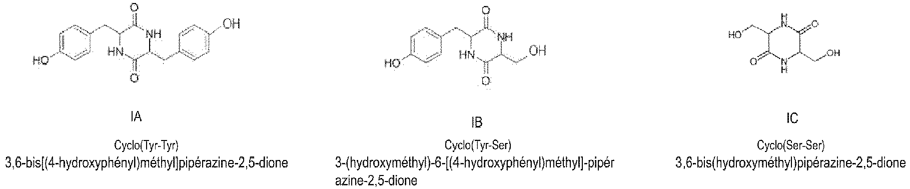 et comprenant en outre les étapes consistant à :
α) faire réagir, en présence d'au moins une base, ledit composé de Formule générale IA, IB ou IC avec un composé ester thiolate ayant la Formule générale VII : 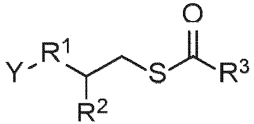 dans lequel :
Y désigne un halogène ;
R¹ est un groupe alkylène en C₁ à C₁₈, un groupe oxyalkylène en C₂ à C₁₈, un groupe thioalkylène en C₂ à C₁₈, des groupes arylène en C₆ à C₁₈ ou aralkylène en C₇ à C₁₈, dans lequel les groupes alkylène, le groupe oxyalkylène et le groupe thioalkylène peuvent être non substitués ou peuvent être substitués avec un halogène ou hydroxyle ou plus ; et les groupes arylène peuvent être non substitués ou peuvent être substitués avec un halogène ou plus ;
R² est H ou méthyle ; et,
R³ est alkyle en C₁ à C₆, aryle en C₆ à C₁₈, alkylaryle en C₇ à C₁₈ ou aralkyle en C₇ à C₁₈, dans lequel les groupes alkyle et aryle peuvent être non substitués ou peuvent être substitués avec un halogène ou plus, pour former, respectivement, un composé thioester de Formule VA, VB ou VC : 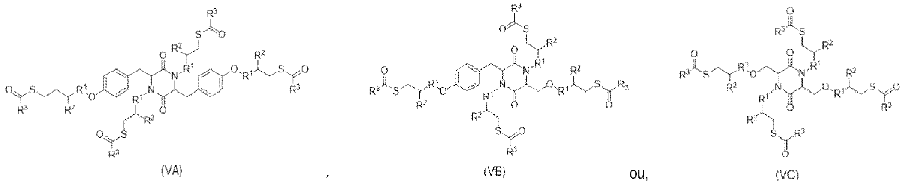
et,
β) déprotéger ledit composé thioester de Formule VA, VB ou VC pour former, respectivement, ledit composé polythiol de Formule VIA, VIB ou VIC.

13. Procédé selon l'une quelconque des revendications 4 à 12, dans lequel ladite étape de déprotection (c), β)) est mise en oeuvre par hydrolyse catalysée par un acide ou catalysée par une base.

14. Procédé selon la revendication 13, dans lequel ledit catalyseur acide ou basique est présent dans ladite étape de déprotection (c), β)) en une quantité allant de 0,05 à 0,25 mole par mole dudit thioester de Formule VA, VB ou VC.

15. Procédé selon la revendication 13 ou la revendication 14, dans lequel ladite étape de déprotection (c), β)) est mise en oeuvre par hydrolyse catalysée par une base et dans lequel en outre ladite base est choisie dans le groupe constitué par : pyridine ; diméthylaminopyridine (DMAP) ; proline ; triazabicyclodécène (TBD) ; diazabicyclo-undécène (DBU) ; hexahydro-méthyl-pyrimido-pyridine (MTBD) ; diazabicyclononane (DBN) ; tétraméthylguanidine (TMG) ; et, triéthylènediamine (TED, *1,4-diazabicyclo[2.2.2]octane).*

16. Procédé selon l'une quelconque des revendications 4 à 15, dans lequel ledit
ledit composé de Formule IA est fourni par l'homocyclisation de tyrosine ;
ledit composé de Formule IB est fourni par l'hétérocyclisation de tyrosine et de sérine ; et,
ledit composé de Formule IC est fourni par l'homocyclisation de sérine.

17. Utilisation du composé polythiol selon l'une quelconque des revendications 1 à 3 en tant que composant réactif d'une composition durcissable à un (1K) ou deux (2K) composants.

18. Composition durcissable comprenant :
c) au moins un polythiol selon l'une quelconque des revendications 1 à 3 ; et,
d) au moins un composé réactif au thiol, dans lequel le ou chaque composé réactif au thiol précité a au moins un groupe fonctionnel (F) choisi dans le groupe constitué par : groupes époxyde ; groupes oxétane ; groupes carbonate cyclique ; groupes anhydride cyclique ; groupes 1-oxacycloalcan-2-one ; groupes à insaturation éthylénique ; groupes alcyne ; et, groupes isocyanate, dans laquelle ledit composé réactif au thiol b) est de préférence choisi dans le groupe constitué par, sans caractère limitatif : composés époxyde ; composés à insaturation éthylénique ; composés (méth)acrylate d'époxy ayant au moins un groupe (méth)acrylate et au moins un groupe époxyde ; et, polyisocyanates.
